# EUROPEAN PATENT APPLICATION

(11) **EP 4 834 882 A1**
(43) Date of publication of application: **04.11.2026**
(21) Application number: 24911330.9
(22) Date of filing: 26.12.2024
(51) Int. Cl.: C07D 221/04, C07D 401/04, A61K 31/444, A61K 31/4709, A61K 31/4725, A61P 3/12, A61P 9/12

(54) **ALDOSTERONE SYNTHETASE INHIBITOR, AND PREPARATION METHOD THEREFOR AND USE THEREOF**

(30) Priority: 29.12.2023 CN 202311867572; 04.03.2024 CN 202410246961; 31.05.2024 CN 202410702699
(71) Applicant: Shenzhen Salubris Pharmaceuticals Co., Ltd., Shenzhen, Guangdong 518017 (CN)
(72) Inventor: WU, Junjun, Shenzhen, Guangdong 518017 (CN); LU, Yinsuo, Shenzhen, Guangdong 518017 (CN); YANG, Zhao, Shenzhen, Guangdong 518017 (CN); XIAO, Ying, Shenzhen, Guangdong 518017 (CN); LI, Hongchao, Shenzhen, Guangdong 518017 (CN); SONG, Zenan, Shenzhen, Guangdong 518017 (CN); YANG, Yang, Shenzhen, Guangdong 518017 (CN)
(74) Representative: karo IP
(86) International application number: PCT/CN2024/142727
(87) International publication number: WO 2025/140409

(57) **Abstract**

The present invention belongs to the technical field of chemical pharmaceuticals. Provided in the present invention are an aldosterone synthase inhibitor, and a preparation method therefor and the use thereof.

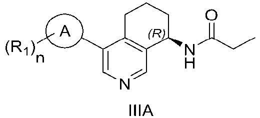

## Description

### TECHNICAL FIELD

The present invention belongs to the technical field of chemical pharmaceuticals. Provided in the present invention are an aldosterone synthase inhibitor, and a preparation method therefor and a use thereof.

### BACKGROUND ART

Aldosterone is a steroid hormone with mineralocorticoid activity, primarily produced by the zona glomerulosa of the adrenal cortex in response to angiotensin II, adrenocorticotropic hormone (ACTH), and increased serum potassium levels. The main physiological role of aldosterone in the kidney is to maintain sodium and potassium balance by regulating cation exchange (Na+ reabsorption and K+ secretion) in the distal nephron. However, aldosterone has also been shown to be a pro-inflammatory and pro-fibrotic hormone in blood vessel, heart and kidney. The effect of aldosterone on gene expression is mediated through binding to the mineralocorticoid receptor (MR) and the typical nuclear hormone receptor pathway.

CYP11B2 (aldosterone synthase) is a cytochrome P450 enzyme, known as an enzyme that catalyzes a series of reactions from 11-deoxycorticosterone (i.e., aldosterone precursor) to aldosterone. CYP11B2 is mainly expressed in the zona glomerulosa of the adrenal cortex, and plasma aldosterone is regulated by the activity of this enzyme in the adrenal gland. In addition, the expression of aldosterone has also been confirmed in sites other than the adrenal gland, such as the cardiovascular system, kidney, adipose tissue, brain, etc., and findings that aldosterone produced locally in various organs is related to organ dysfunction have attracted attention. It has been reported that a CYP11B2 inhibitor is capable of inhibiting aldosterone production in studies using enzyme(s) and cultured cells, and has the effect of inhibiting aldosterone production and therapeutic effects in studies using various experimental animal models. Furthermore, it has been confirmed that a CYP11B2 inhibitor shows effects of reducing plasma and urinary aldosterone levels and antihypertensive effects in patients with hypertension and patients with primary aldosteronism. Finding means to block the biosynthetic pathway of aldosterone is a highly achievable approach for establishing effective treatment methods for various diseases related to aldosterone.

Currently, aldosterone synthase (CYP11B2) inhibitors have been reported in the prior art, for example, the patent CN103827101B discloses a bicyclic dihydroquinolin-2-one derivative, which, although possessing good CYP11B2 inhibitory activity, suffers from poor selectivity;
The patent application CN114853755A also reports an aldosterone synthase inhibitor. However, this compound is chiral, making its synthesis relatively difficult, and it is also associated with significant side effects. Furthermore, existing CYP11B2 inhibitors also have problems such as poor activity, difficult synthesis, poor stability, and low bioavailability. Therefore, there is an urgent need in the art to provide more CYP11B2 inhibitors.

### SUMMARY OF INVENTION

In view of the problems existing in the prior art, the present invention provides an aldosterone synthase inhibitor, a preparation method therefor and a use thereof, so as to solve the problems existing in the prior art.

The present invention is achieved through the following embodiments:
The present invention provides an aldosterone synthase inhibitor, an isomer, a racemate, or a pharmaceutically acceptable salt thereof, wherein the structure of the aldosterone synthase inhibitor is as shown in Formula I or Formula IA: 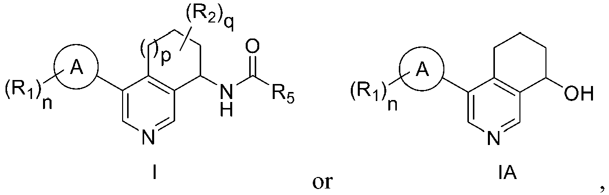 wherein,
each of R₁ or R₂ is independently selected from H, halogen, hydroxyl, cyano, substituted or unsubstituted C₁-C₈ alkyl, substituted or unsubstituted C₁-C₈ alkoxy, substituted or unsubstituted C₃-C₈ cycloalkyl, substituted or unsubstituted 3-8 membered heterocycloalkyl, C₁-C₆ alkylsulfonyl, substituted or unsubstituted C₆-C₁₂ aryl or substituted or unsubstituted 5-12 membered heteroaryl; R₅ is independently selected from substituted or unsubstituted C₁-C₈ alkyl, substituted or unsubstituted C₁-C₈ alkoxy, substituted or unsubstituted C₃-C₈ cycloalkyl, substituted or unsubstituted 3-8 membered heterocycloalkyl, substituted or unsubstituted C₆-C₁₂ aryl or substituted or unsubstituted 5-12 membered heteroaryl;
ring A is independently selected from 5-12 membered fused heteroaryl or C₁₀-C₁₂ aryl;
the substituents in the above "substituted" are each independently selected from one or more of C₁-C₈ alkyl, halo C₁-C₈ alkyl, C₁-C₈ alkoxy, halo C₁-C₈ alkoxy, -NR₃R₄, hydroxyl, oxo, carboxyl, cyano, halogen, C₁-C₆ alkylsulfonyl, C₁-C₆ alkylacyl, C₁-C₆ alkylamido, C₃-C₈ cycloalkyl, C₃-C₈ cycloalkyloxy, 3-8 membered heterocycloalkyl, 3-8 membered heterocycloalkyloxy, C₃-C₈ cycloalkyl-substituted C₁-C₈ alkoxy, C₆-C₁₂ aryl, C₆-C₁₂ aryloxy, C₁-C₈ alkyl-substituted C₆-C₁₂ aryloxy, C₁-C₈ alkoxy-substituted C₆-C₁₂ aryloxy, halo C₁-C₈ alkyl-substituted C₆-C₁₂ aryloxy, 5-12 membered heteroaryl, 5-12 membered heteroaryloxy, C₁-C₈ alkyl-substituted 5-12 membered heteroaryloxy, C₁-C₈ alkoxy-substituted 5-12 membered heteroaryloxy or halo C₁-C₈ alkyl-substituted 5-12 membered heteroaryloxy;
R₃ and R₄ are each independently selected from H or C₁-C₈ alkyl;
n, p or q are each independently selected from an integer of 0, 1, 2 or 3.

Further, as a preferable embodiment of the present invention, the present invention also provides an aldosterone synthase inhibitor, an isomer, a racemate, or a pharmaceutically acceptable salt thereof, wherein the structure of the aldosterone synthase inhibitor is as shown in Formula IB: 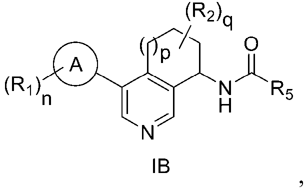 wherein,
each of R₁ or R₂ is independently selected from H, halogen, cyano, substituted or unsubstituted C₁-C₈ alkyl, substituted or unsubstituted C₁-C₈ alkoxy, substituted or unsubstituted C₃-C₈ cycloalkyl, substituted or unsubstituted 3-8 membered heterocycloalkyl, substituted or unsubstituted C₆-C₁₂ aryl or substituted or unsubstituted 5-12 membered heteroaryl;
R₅ is independently selected from substituted or unsubstituted C₁-C₈ alkyl, substituted or unsubstituted C₁-C₈ alkoxy, substituted or unsubstituted C₃-C₈ cycloalkyl, substituted or unsubstituted 3-8 membered heterocycloalkyl, substituted or unsubstituted C₆-C₁₂ aryl or substituted or unsubstituted 5-12 membered heteroaryl;
ring A is independently selected from 5-12 membered fused heteroaryl;
the substituents in the above "substituted" are each independently selected from one or more of C₁-C₈ alkyl, halo C₁-C₈ alkyl, C₁-C₈ alkoxy, halo C₁-C₈ alkoxy, -NR₃R₄, hydroxyl, oxo, carboxyl, cyano, halogen, C₁-C₆ alkylsulfonyl, C₁-C₆ alkylamido, C₃-C₈ cycloalkyl, C₃-C₈ cycloalkyloxy, 3-8 membered heterocycloalkyl, 3-8 membered heterocycloalkyloxy, C₃-C₈ cycloalkyl-substituted C₁-C₈ alkoxy, C₆-C₁₂ aryl, C₆-C₁₂ aryloxy, C₁-C₈ alkyl-substituted C₆-C₁₂ aryloxy, C₁-C₈ alkoxy-substituted C₆-C₁₂ aryloxy, halo C₁-C₈ alkyl-substituted C₆-C₁₂ aryloxy, 5-12 membered heteroaryl, 5-12 membered heteroaryloxy, C₁-C₈ alkyl-substituted 5-12 membered heteroaryloxy, C₁-C₈ alkoxy-substituted 5-12 membered heteroaryloxy or halo C₁-C₈ alkyl-substituted 5-12 membered heteroaryloxy;
R₃ and R₄ are each independently selected from H or C₁-C₈ alkyl;
n, p or q are each independently selected from an integer of 0, 1, 2 or 3.

Further, as a preferable embodiment of the present invention, each of R₁ or R₂ is independently selected from H, halogen, cyano, substituted or unsubstituted C₁-C₆ alkyl, substituted or unsubstituted C₁-C₆ alkoxy, substituted or unsubstituted C₃-C₈ cycloalkyl, substituted or unsubstituted 3-8 membered heterocycloalkyl, substituted or unsubstituted C₆-C₁₀ aryl or substituted or unsubstituted 5-10 membered heteroaryl;
R₅ is independently selected from substituted or unsubstituted C₁-C₆ alkyl, substituted or unsubstituted C₁-C₆ alkoxy, substituted or unsubstituted C₃-C₈ cycloalkyl, substituted or unsubstituted 3-8 membered heterocycloalkyl, substituted or unsubstituted C₆-C₁₀ aryl or substituted or unsubstituted 5-10 membered heteroaryl;
ring A is independently selected from 6-10 membered fused heteroaryl;
the substituents in the above "substituted" are each independently selected from one or more of C₁-C₆ alkyl, halo C₁-C₆ alkyl, C₁-C₆ alkoxy, halo C₁-C₆ alkoxy, -NR₃R₄, hydroxyl, oxo, carboxyl, cyano, halogen, C₁-C₆ alkylsulfonyl, C₁-C₆ alkylamido, C₃-C₈ cycloalkyl, C₃-C₈ cycloalkyloxy, 3-8 membered heterocycloalkyl, 3-8 membered heterocycloalkyloxy, C₃-C₈ cycloalkyl-substituted C₁-C₆ alkoxy, C₆-C₁₀ aryl, C₆-C₁₀ aryloxy, C₁-C₆ alkyl-substituted C₆-C₁₀ aryloxy, C₁-C₆ alkoxy-substituted C₆-C₁₀ aryloxy, halo C₁-C₆ alkyl-substituted C₆-C₁₀ aryloxy, 5-10 membered heteroaryl, 5-10 membered heteroaryloxy, C₁-C₆ alkyl-substituted 5-10 membered heteroaryloxy, C₁-C₆ alkoxy-substituted 5-10 membered heteroaryloxy or halo C₁-C₆ alkyl-substituted 5-10 membered heteroaryloxy;
R₃ and R₄ are each independently selected from H or C₁-C₆ alkyl;
n, p or q are each independently selected from an integer of 0, 1, 2 or 3.

Further, as a preferable embodiment of the present invention, each of R₁ or R₂ is independently selected from H, halogen, cyano, substituted or unsubstituted C₁-C₃ alkyl, substituted or unsubstituted C₁-C₃ alkoxy, substituted or unsubstituted C₃-C₆ cycloalkyl, substituted or unsubstituted 3-6 membered heterocycloalkyl, substituted or unsubstituted C₆-C₈ aryl or substituted or unsubstituted 5-8 membered heteroaryl;
R₅ is independently selected from substituted or unsubstituted C₁-C₃ alkyl, substituted or unsubstituted C₁-C₃ alkoxy, substituted or unsubstituted C₃-C₆ cycloalkyl, substituted or unsubstituted 3-6 membered heterocycloalkyl, substituted or unsubstituted C₆-C₈ aryl or substituted or unsubstituted 5-8 membered heteroaryl;
ring A is independently selected from 6-9 membered fused heteroaryl;
the substituents in the above "substituted" are each independently selected from one or more of C₁-C₃ alkyl, halo C₁-C₃ alkyl, C₁-C₃ alkoxy, halo C₁-C₃ alkoxy, -NR₃R₄, hydroxyl, oxo, carboxyl, cyano, halogen, C₁-C₃ alkylsulfonyl, C₁-C₃ alkylamido, C₃-C₆ cycloalkyl, C₃-C₆ cycloalkyloxy, 3-6 membered heterocycloalkyl, 3-6 membered heterocycloalkyloxy, C₃-C₆ cycloalkyl-substituted C₁-C₃ alkoxy, C₆-C₈ aryl, C₆-C₈ aryloxy, C₁-C₃ alkyl-substituted C₆-C₈ aryloxy, C₁-C₃ alkoxy-substituted C₆-C₈ aryloxy, halo C₁-C₃ alkyl-substituted C₆-C₈ aryloxy, 5-8 membered heteroaryl, 5-8 membered heteroaryloxy, C₁-C₃ alkyl-substituted 5-8 membered heteroaryloxy, C₁-C₃ alkoxy-substituted 5-8 membered heteroaryloxy or halo C₁-C₃ alkyl-substituted 5-8 membered heteroaryloxy;
R₃ and R₄ are each independently selected from H or C₁-C₃ alkyl;
n, p or q are each independently selected from an integer of 0, 1, 2 or 3.

Further, the present invention also provides an aldosterone synthase inhibitor, an isomer, a racemate, or a pharmaceutically acceptable salt thereof, wherein the structure of the aldosterone synthase inhibitor is as shown in Formula IIA or Formula IIB: 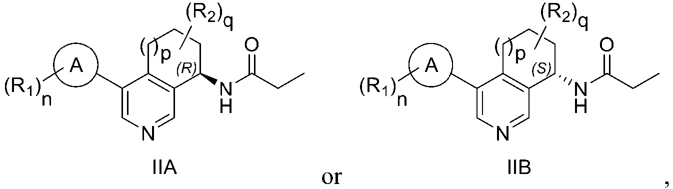 wherein, the definitions of R₁, R₂, ring A, n, p and q are the same as defined above.

Further, the present invention also provides an aldosterone synthase inhibitor, an isomer, a racemate, or a pharmaceutically acceptable salt thereof, wherein the structure of the aldosterone synthase inhibitor is as shown in Formula IIIA, Formula IIIB, Formula IIIC or Formula IIID: 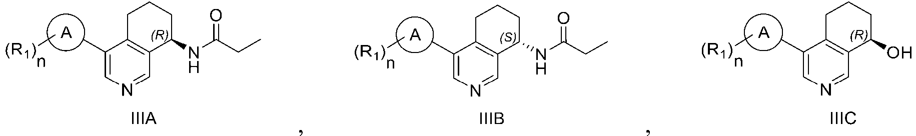 or 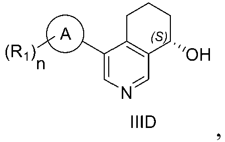 wherein, the definitions of the R₁, R₂, ring A, n, p and q are the same as defined above.

Further, as a preferable embodiment of the present invention, ring A is selected from: 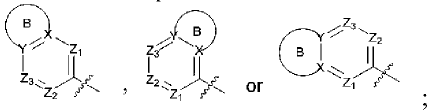 preferably, ring A is selected from: 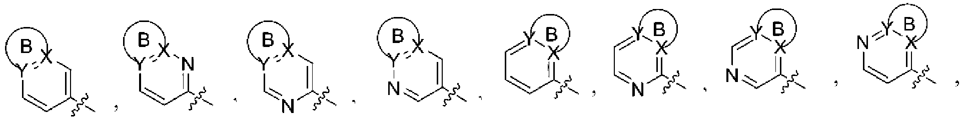 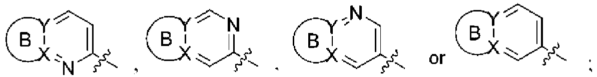 , wherein, ring B is selected from 5-10 membered heteroaryl or C₆-C₁₀ aryl; X, Y, Z₁, Z₂ or Z₃ are each independently selected from C or N.

Further, as a preferable embodiment of the present invention, ring A is selected from 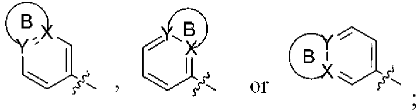 wherein, ring B is selected from 5-10 membered heteroaryl; X or Y is independently selected from C or N.

Further, as a preferable embodiment of the present invention, ring A is selected from 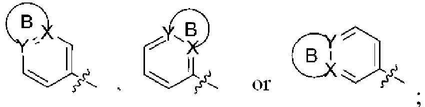 wherein, ring B is selected from 5-10 membered heteroaryl; X or Y is independently selected from C or N.

Further, as a preferable embodiment of the present invention, ring B is selected from 5-10 membered heteroaryl, preferably 5-8 membered heteroaryl; more preferably 5-6 membered heteroaryl. Examples of ring B include but are not limited to: pyrrolyl, furanyl, thienyl, imidazolyl, oxazolyl, thiazolyl, triazolyl, oxadiazolyl, thiadiazolyl, tetrazolyl, pyridyl, pyrazinyl, pyrazolyl, pyridazinyl, triazinyl, isoxazolyl, or isothiazolyl.

Further, as a preferable embodiment of the present invention, ring A is selected from benzothienyl, benzofuranyl, indolyl, isoindolyl, benzoxazolyl, benzimidazolyl, benzothiazolyl, benzoisoxazolyl, isobenzofuranyl, benzothiazolyl, benzoisothiazolyl, benzoxadiazolyl, benzothiadiazolyl, benzotriazolyl, purinyl, indazolyl, quinolinyl, isoquinolinyl, quinazolinyl, quinoxalinyl, benzopyridazinyl, imidazopyridyl, naphthyridinyl, or naphthyl.

Further, as a preferable embodiment of the present invention, ring A is selected from: 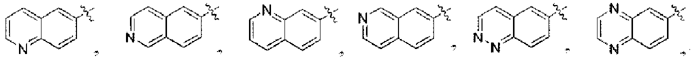 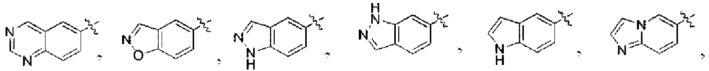 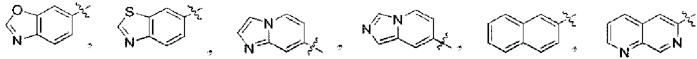 or

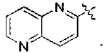

Further, as a preferable embodiment of the present invention, each of R₁ or R₂ is independently selected from H, hydroxyl, halogen, cyano, C₁-C₆ alkyl, halo C₁-C₆ alkyl, C₁-C₆ alkoxy, halo C₁-C₆ alkoxy, C₃-C₈ cycloalkyl, C₃-C₈ cycloalkoxy, C₁-C₆ alkylsulfonyl, C₁-C₆ alkylacyl, 3-8 membered heterocycloalkyl, C₆-C₁₀ aryl or 5-10 membered heteroaryl; further, as a preferable embodiment of the present invention, each of R₁ or R₂ is independently selected from H, halogen, cyano, C₁-C₃ alkyl, halo C₁-C₃ alkyl, C₁-C₃ alkoxy, halo C₁-C₃ alkoxy, C₃-C₈ cycloalkyl, 3-8 membered heterocycloalkyl, C₆-C₈ aryl or 5-8 membered heteroaryl.

Further, as a preferable embodiment of the present invention, each of R₁ or R₂ is independently selected from H, hydroxyl, F, Cl, cyano, methyl, ethyl, propyl, isopropyl, methoxy, ethoxy, trifluoromethyl, trifluoroethyl, trifluoromethoxy, trifluoroethoxy, cyclopropyl, cyclopropyloxy, methylsulfonyl, ethylsulfonyl, formyl or acetyl.

Further, as a preferable embodiment of the present invention, each of R₁ or R₂ is independently selected from H, F, Cl, cyano, methyl, methoxy, or trifluoromethyl.

As a preferable embodiment of the present invention, the C₁-C₈ alkyl is preferably C₁-C₂, C₁-C₃, C₁-C₄, C₁-C₅ or C₁-C₆ alkyl. Examples of the alkyl include methyl, ethyl, propyl, isopropyl, n-butyl, isobutyl, sec-butyl, tert-butyl, n-pentyl, sec-pentyl, 1-ethylpropyl, 2-methylbutyl, tert-pentyl, 1,2-dimethylpropyl, isopentyl, neopentyl, n-hexyl, isohexyl, sec-hexyl, tert-hexyl, neohexyl, 2-methylpentyl, 1,2-dimethylbutyl, and 1-ethylbutyl.

As a preferable embodiment of the present invention, the C₁-C₈ alkoxy is preferably C₁-C₂, C₁-C₃, C₁-C₄, C₁-C₅ or C₁-C₆ alkoxy. Further, examples of the alkoxy include methoxy, ethoxy, propoxy, isopropoxy, n-butoxy, isobutoxy, sec-butoxy, tert-butoxy.

As a preferable embodiment of the present invention, the C₃-C₈ cycloalkyl is preferably selected from C₃-C₆ cycloalkyl or C₃-C₅ cycloalkyl. The cycloalkyl is specifically selected from cyclopropyl, cyclobutyl, cyclopentyl, cyclohexyl, cycloheptyl and cyclooctyl.

As a preferable embodiment of the present invention, the C₃-C₈ cycloalkoxy is preferably selected from C₃-C₆ cycloalkoxy or C₃-C₅ cycloalkoxy. The cycloalkoxy is specifically selected from cyclopropoxy, cyclobutoxy, cyclopentoxy, cyclohexoxy, cycloheptoxy and cyclooctoxy.

As a preferable embodiment of the present invention, the heterocycloalkyl is selected from 3-12 membered heterocycloalkyl, preferably 3-10 membered heterocycloalkyl, 3-8 membered heterocycloalkyl, 3-6 membered heterocycloalkyl or 3-5 membered heterocycloalkyl. Examples of the heterocycloalkyl include aziridinyl, oxiranyl, azetidinyl, oxetanyl, pyrrolidinyl, tetrahydrofuranyl, tetrahydro-thienyl, pyrazolidinyl, imidazolidinyl, oxazolidinyl, isoxazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, oxazepanyl and thiazinanyl, 8-aza-bicyclo[3.2.1]octyl, quinuclidinyl, 8-oxa-3-aza-bicyclo[3.2.1]octyl, 9-aza-bicyclo[3.3.1]nonyl, 3-oxa-9-aza-bicyclo[3.3.1]nonyl, 3-thia-9-aza-bicyclo[3.3.1]nonyl and 2,6-diaza-spiro[3.3]heptanyl. Examples of partially unsaturated heterocycloalkyl are dihydrofuranyl, imidazolinyl, dihydro-oxazolyl, tetrahydropyridinyl, or dihydropyranyl; preferable examples of heterocycloalkyl are pyrrolidinyl, pyrazolidinyl, imidazolidinyl, oxazolidinyl, isoxazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, oxazepanyl, thiazinanyl and 2,6-diaza-spiro[3.3]heptanyl. More preferable examples of heterocycloalkyl are pyrrolidinyl, piperidinyl, thiomorpholinyl, thiazinanyl and 2,6-diaza-spiro[3.3]heptanyl.

As a preferable embodiment of the present invention, the halo C₁-C₈ alkyl or C₁-C₈ haloalkyl is preferably halo C₁-C₂, C₁-C₃, C₁-C₄, C₁-C₅ or C₁-C₆ alkyl or C₁-C₂, C₁-C₃, C₁-C₄, C₁-C₅ or C₁-C₆ haloalkyl. Examples of the haloalkyl include fluoromethyl, difluoromethyl, trifluoromethyl, trifluoroethyl, trifluoromethylethyl and pentafluoroethyl, and the particularly preferred haloalkyls are trifluoromethyl and trifluoroethyl.

As a preferable embodiment of the present invention, the substituted or unsubstituted C₆-C₁₂ aryl is preferably substituted or unsubstituted C₆-C₁₀ aryl, substituted or unsubstituted C₆-C₈ aryl, substituted or unsubstituted C₆-C₁₂ aryl. Examples of the substituted or unsubstituted aryl include phenyl, o-tolyl, m-tolyl, p-tolyl, phenolyl, xylyl, chlorophenyl, dichlorophenyl, nitrophenyl, cyanophenyl or naphthyl.

As a preferable embodiment of the present invention, the substituted or unsubstituted 5-12 membered heteroaryl is preferably substituted or unsubstituted 5-10 membered heteroaryl, substituted or unsubstituted 5-8 membered heteroaryl, substituted or unsubstituted 5-8 membered heteroaryl, substituted or unsubstituted 5-7 membered heteroaryl, substituted or unsubstituted 5-6 membered heteroaryl. Examples of the substituted or unsubstituted heteroaryl include pyrrolyl, furanyl, thienyl, imidazolyl, oxazolyl, thiazolyl, triazolyl, oxadiazolyl, thiadiazolyl, tetrazolyl, pyridyl, pyrazinyl, pyrazolyl, pyridazinyl, pyrimidinyl, triazinyl, azepinyl, diazepinyl, isoxazolyl, benzofuranyl, isothiazolyl, benzothienyl, indolyl, isoindolyl, isobenzofuranyl, benzimidazolyl, benzoxazolyl, benzoisoxazolyl, benzothiazolyl, benzoisothiazolyl, benzoxadiazolyl, benzothiadiazolyl, benzotriazolyl, purinyl, quinolinyl, isoquinolinyl, quinazolinyl and quinoxalinyl. Particularly, the heteroaryl include pyrrolyl, furanyl, thienyl, imidazolyl, oxazolyl, thiazolyl, triazolyl, oxadiazolyl, thiadiazolyl, tetrazolyl, pyridyl, pyrazinyl, pyrazolyl, pyridazinyl, pyrimidinyl, isoxazolyl and isothiazolyl. More particularly, the heteroaryl include imidazolyl, oxazolyl, thiazolyl, oxadiazolyl, thiadiazolyl, tetrazolyl, pyridyl, pyrazinyl, pyrazolyl, pyridazinyl, pyrimidinyl, indazolyl, isoxazolyl and isothiazolyl.

As a preferable embodiment of the present invention, the 5-12 membered fused heteroaryl is preferably 5-10 membered fused heteroaryl, 6-10 membered fused heteroaryl, 6-9 membered fused heteroaryl, 6-8 membered fused heteroaryl; examples of the fused heteroaryl include, but are not limited to: benzothienyl, benzofuranyl, indolyl, isoindolyl, benzoxazolyl, benzimidazolyl, benzothiazolyl, benzoisoxazolyl, isobenzofuranyl, benzothiazolyl, benzoisothiazolyl, benzoxadiazolyl, benzothiadiazolyl, benzotriazolyl, purinyl, indazolyl, quinolinyl, isoquinolinyl, quinazolinyl, quinoxalinyl, benzopyridazinyl, imidazopyridyl, naphthyridinyl, or naphthyl.

As a preferable embodiment of the present invention, the C₆-C₁₂ aryl is preferably C₆-C₁₀ aryl or C₁₀-C₁₂ aryl; the C₆-C₁₀ aryl is preferably C₆-C₈ aryl, more preferably phenyl; the C₁₀-C₁₂ aryl is more preferably naphthyl.

As a preferable embodiment of the present invention, halogen is selected from fluorine, chlorine, bromine, or iodine.

As a preferable embodiment of the present invention, the aldosterone synthase inhibitor, or an isomer thereof, or a racemate thereof, or a pharmaceutically acceptable salt thereof is selected from

| No. | Structure | No. | Structure | No. | Structure |
|---|---|---|---|---|---|
| 1 | 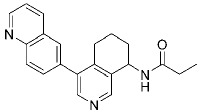 | 1A | 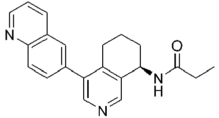 | 1B | 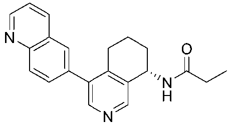 |
| 2 | 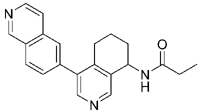 | 2A | 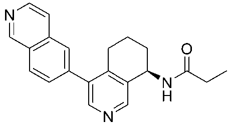 | 2B | 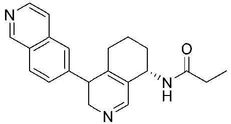 |
| 3 | 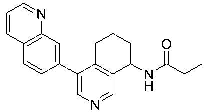 | 3A | 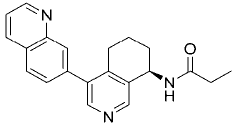 | 3B | 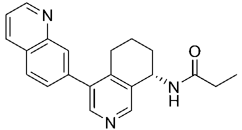 |
| 4 | 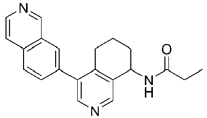 | 4A | 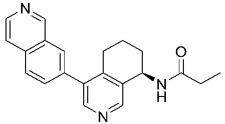 | 4B | 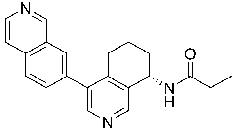 |
| 5 | 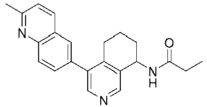 | 5A | 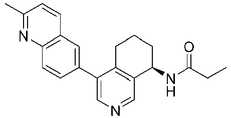 | 5B | 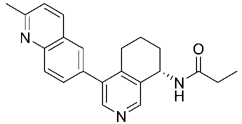 |
| 6 | 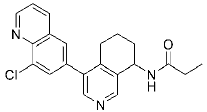 | 6A | 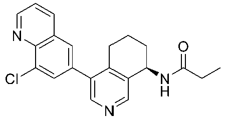 | 6B | 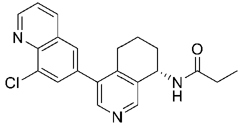 |
| 7 | 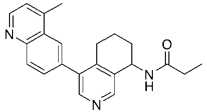 | 7A | 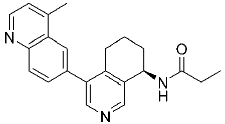 | 7B | 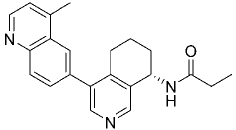 |
| 8 | 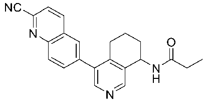 | 8A | 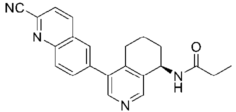 | 8B | 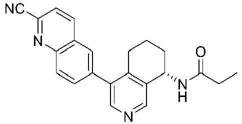 |
| 9 | 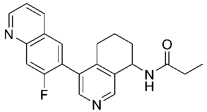 | 9A | 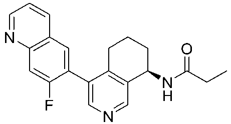 | 9B | 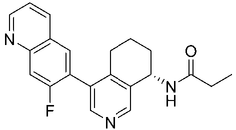 |
| 11 | 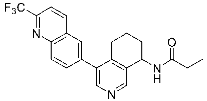 | 11A | 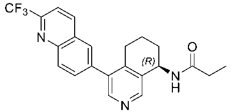 | 11B | 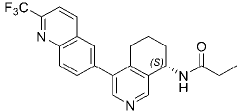 |
| 12 | 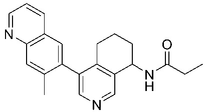 | 12A | 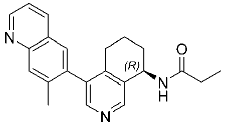 | 12B | 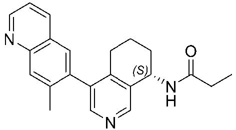 |
| 13 | 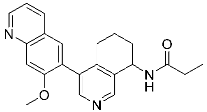 | 13A | 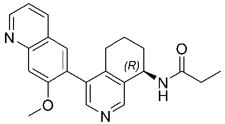 | 13B | 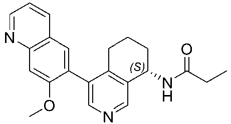 |
| 14 | 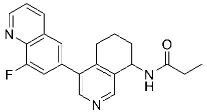 | 14A | 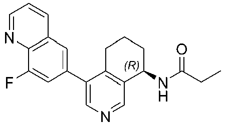 | 14B | 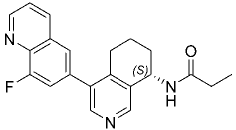 |
| 15 | 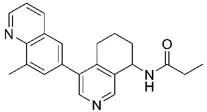 | 15A | 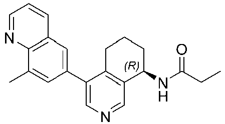 | 15B | 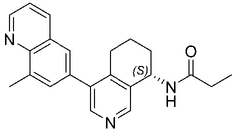 |
| 16 | 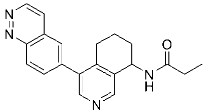 | 16A | 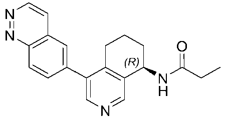 | 16B | 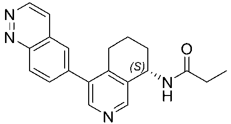 |
| 17 | 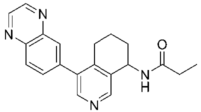 | 17A | 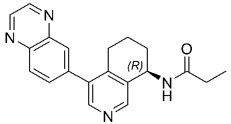 | 17B | 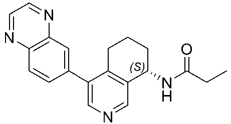 |
| 18 | 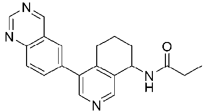 | 18A | 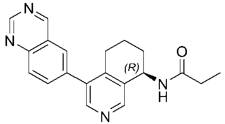 | 18B | 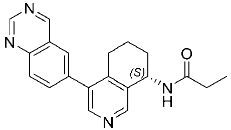 |
| 19 | 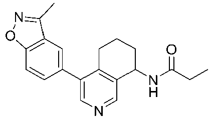 | 19A | 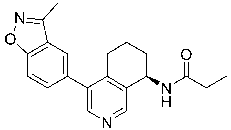 | 19B | 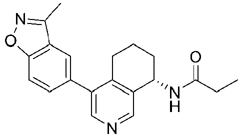 |
| 20 | 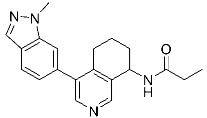 | 20A | 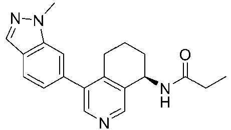 | 20B | 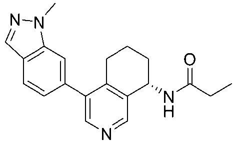 |
| 22 | 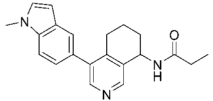 | 22A | 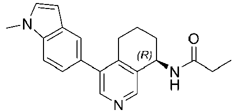 | 22B | 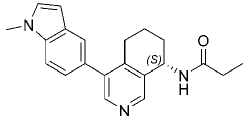 |
| 23 | 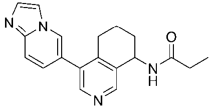 | 23A | 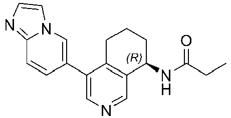 | 23B | 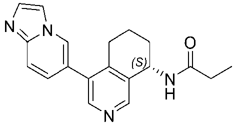 |
| 24 | 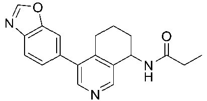 | 24A | 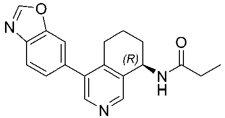 | 24B | 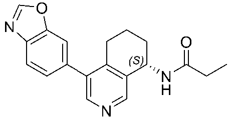 |
| 25 | 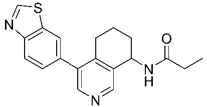 | 25A | 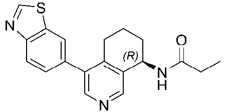 | 25B | 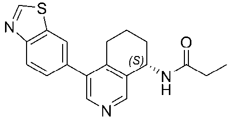 |
| 26 | 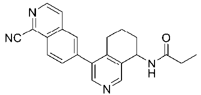 | 26A | 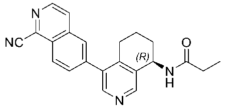 | 26B | 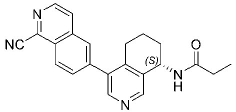 |
| 27 | 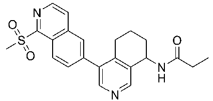 | 27A | 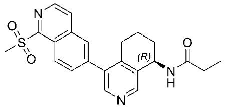 | 27B | 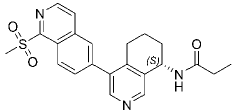 |
| 28 | 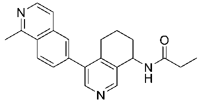 | 28A | 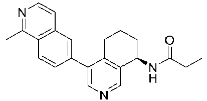 | 28B | 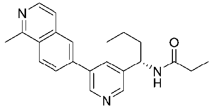 |
| 29 | 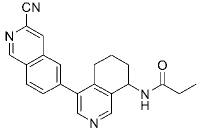 | 29A | 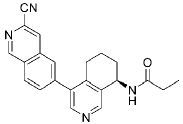 | 29B | 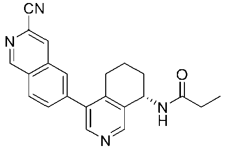 |
| 31 | 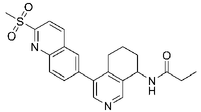 | 31A | 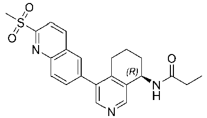 | 31B | 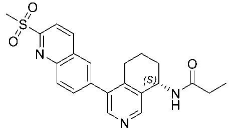 |
| 32 | 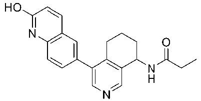 | 32A | 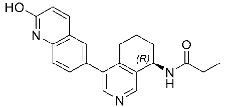 | 32B | 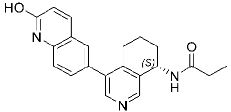 |
| 33 | 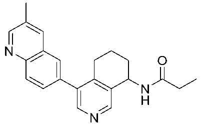 | 33A | 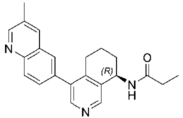 | 33B | 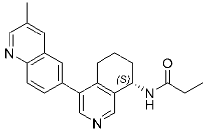 |
| 34 | 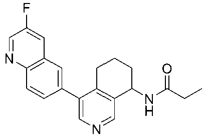 | 34A | 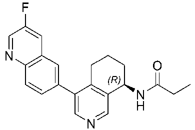 | 34B | 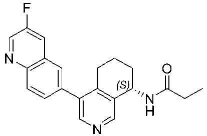 |
| 35 | 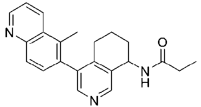 | 35A | 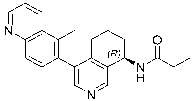 | 35B | 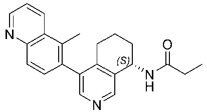 |
| 36 | 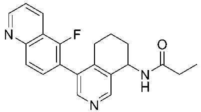 | 36A | 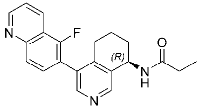 | 36B | 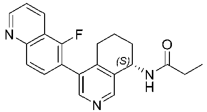 |
| 37 | 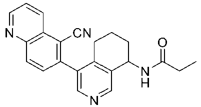 | 37A | 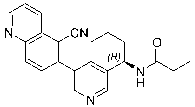 | 37B | 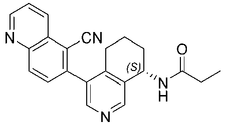 |
| 38 | 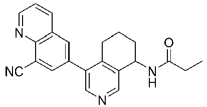 | 38A | 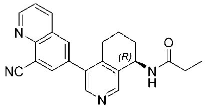 | 38B | 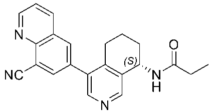 |
| 39 | 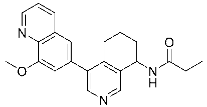 | 39A | 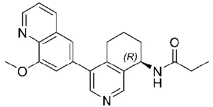 | 39B | 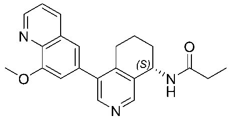 |
| 41 | 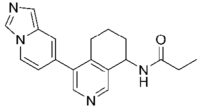 | 41A | 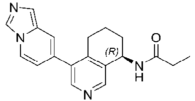 | 41B | 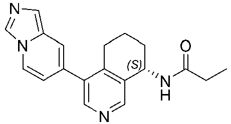 |
| 42 | 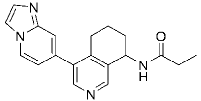 | 42A | 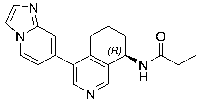 | 42B | 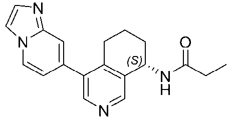 |
| 43 | 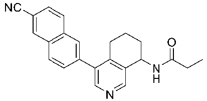 | 43A | 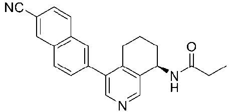 | 43B | 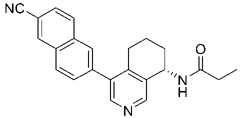 |
| 44 | 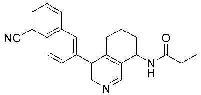 | 44A | 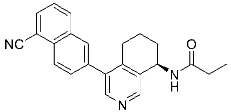 | 44B | 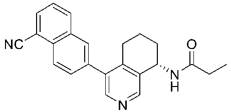 |
| 45 | 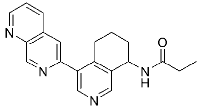 | 45A | 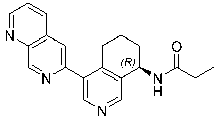 | 45B | 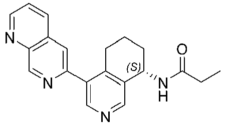 |
| 46 | 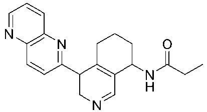 | 46A | 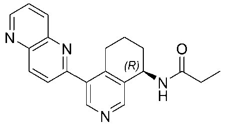 | 46B | 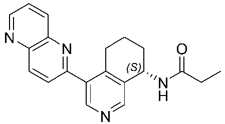 |
| 47 | 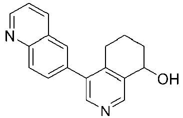 | 47A | 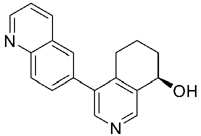 | 47B | 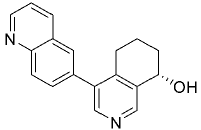 |
| 48 | 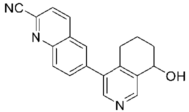 | 48A | 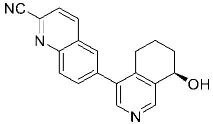 | 48B | 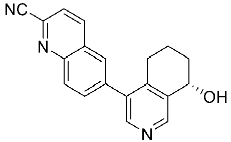 |
| 49 | 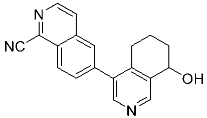 | 49A | 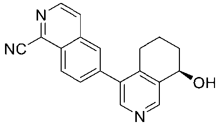 | 49B | 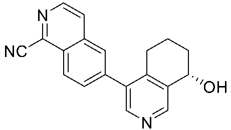 |

The present invention also provides a deuterated compound of the aforementioned aldosterone synthase inhibitor, or an isomer thereof, or a racemate thereof, or a pharmaceutically acceptable salt thereof.

Further, as a preferable embodiment of the present invention, the deuterated compound is selected from the following structures:

| No. | Structure | No. | Structure | No. | Structure |
|---|---|---|---|---|---|
| 40 | 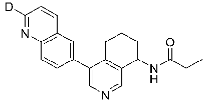 | 40A | 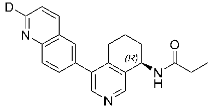 | 40B | 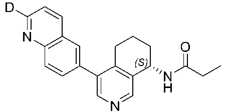 |

The present invention further provides a pharmaceutical composition, comprising the aldosterone synthase inhibitor according to Formula I, Formula IA, Formula IB, Formula IIA, Formula IIB, Formula IIIA, Formula IIIB, Formula IIIC or Formula IIID, an isomer thereof, a racemate thereof, or a pharmaceutically acceptable salt thereof or a deuterated compound thereof, and one or more pharmaceutically acceptable excipients and/or carriers.

The present invention further provides a use of the aldosterone synthase inhibitor according to Formula I, Formula IA, Formula IB, Formula IIA, Formula IIB, Formula IIIA, Formula IIIB, Formula IIIC or formula IIID, an isomer thereof, a racemate thereof, or a pharmaceutically acceptable salt thereof, or a deuterated compound thereof, or the aforementioned pharmaceutical composition, in the preparation of a medicament for treating or preventing diseases associated with elevated activity level of CYP11B2, including chronic kidney disease, congestive heart failure, hypertension, diabetic nephropathy, primary aldosteronism and Cushing's syndrome.

As a preferable embodiment of the present invention, the diseases associated with elevated activity level of CYP11B2 are selected from hypertension, etc.

The present invention further provides a method for preparing an aldosterone synthase inhibitor, an isomer, a racemate, or a pharmaceutically acceptable salt thereof, with reference to patent CN103827101B and conventional methods in the art.

The beneficial effects of the present invention compared to the prior art include but are not limited to:
the aldosterone synthase inhibitor of the present invention can selectively inhibit CYP11B2 while only weakly inhibiting CYP11B1, and exhibits better CYP11B2 inhibitory activity compared to the prior art.

For the sake of clarity, the general terms used in the description of the compounds are generally defined herein.

Unless otherwise stated, the following terms and phrases used herein are intended to have meanings stated below. A specific term or phrase should not be considered as uncertain or unclear without a particular definition, but should be understood according to its ordinary meaning. When a trade name appears herein, it is intended to refer to its corresponding product or its active ingredient. The term "pharmaceutically acceptable" as used herein is directed to those compounds, materials, compositions and/or dosage forms that are, within the scope of sound medical judgment, are suitable for use in contact with the tissues of humans and animals without excessive toxicity, irritation, allergic response, or other problems or complications, commensurate with a reasonable benefit/risk ratio.

The term "pharmaceutically acceptable salt" refers to salts of the compounds of the present invention, prepared from the compounds with specific substituents described herein and pharmaceutically acceptable acids or bases.

In addition to salt forms, the compounds provided by the present invention also exist in prodrug forms. Prodrugs of the compounds described herein readily undergo chemical changes under physiological conditions to convert into the compounds of the present invention. Furthermore, prodrugs can be converted to the compounds of the present invention by chemical or biochemical methods in an in vivo environment.

Certain compounds of the present invention may exist in non-solvated or solvated forms, including hydrated forms. In general, solvated forms are considered equivalent to non-solvated forms. Both are encompassed within the scope of the present invention.

The compounds of the present invention may exist in specific geometric or stereoisomeric forms. The present invention contemplates all such compounds, including cis- and trans-isomers, (-)- and (+)-enantiomers, (R)- and (S)-enantiomers, diastereomers, (D)-isomers, (L)-isomers, and racemic mixtures thereof, and other mixtures, such as enantiomerically or diastereomerically enriched mixtures, all of which are within the scope of the present invention. Additional asymmetric carbon atoms may be present in substituents such as alkyl. All such isomers and mixtures thereof are included within the scope of the present invention.

Optically active (R)- and (S)-isomers, as well as D and L isomers, can be prepared by chiral synthesis or chiral reagents or other conventional techniques. If an specific enantiomer of a compound of the invention is desired, it may be prepared by asymmetric synthesis or derivatization with chiral auxiliary groups, wherein the resulting diastereomeric mixture is separated and the auxiliary groups cleaved to provide the pure desired enantiomer. Alternatively, when the molecule contains a basic functional group (such as amino) or an acidic functional group (such as carboxyl), diastereomeric salts are formed with an appropriate optically active acid or base, followed by resolution of the diastereomers by conventional methods well-known in the art, and then recovery of the pure enantiomer. Furthermore, separation of enantiomers and diastereomers is frequently accomplished using chromatography employing chiral stationary phases, optionally in combination with chemical derivatization (e.g., formation of carbamates from amines).

The term "alkyl" refers to a saturated aliphatic hydrocarbon group, including straight and branched chain groups of 1 to 20 carbon atoms. Preferably, alkyl contains 1 to 8 carbon atoms, more preferably 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, and various branched isomers thereof, etc.

The term "haloalkyl" means an alkyl group in which at least one of the hydrogen atoms has been replaced by a same or different halogen atoms. Examples of haloalkyl include fluoromethyl, difluoromethyl, trifluoromethyl, trifluoroethyl, trifluoromethylethyl and pentafluoroethyl. Particularly, the haloalkyl groups are trifluoromethyl and trifluoroethyl.

The term "alkoxy" refers to -O-(alkyl), wherein alkyl is as defined above. Examples of alkoxy include methoxy, ethoxy, n-propoxy, isopropoxy, n-butoxy, isobutoxy and tert-butoxy. Particularly, the alkoxy include methoxy, ethoxy and tert-butoxy.

The term "halogen" refers to fluorine, chlorine, bromine or iodine.

The term "haloalkoxy" means an alkoxy group in which at least one of the hydrogen atoms of the alkoxy group has been replaced by a same or different halogen atom. The term "perhaloalkoxy" means an alkoxy group in which all hydrogen atoms of the alkoxy group have been replaced by a same or different halogen atoms. Examples of haloalkoxy include fluoromethoxy, difluoromethoxy, trifluoromethoxy, trifluoroethoxy, trifluoromethylethoxy, trifluorodimethylethoxy and pentafluoroethoxy. Particularly, the haloalkoxy are trifluoromethoxy and 2,2-difluoroethoxy.

The term "cycloalkyl" or "carbocycle" refers to a saturated monocyclic or polycyclic cyclic hydrocarbon substituent, wherein the cycloalkyl ring contains 3 to 10 carbon atoms, preferably 3 to 8 carbon atoms, more preferably 3 to 6 carbon atoms. Non-limiting examples of monocyclic cycloalkyl include cyclopropyl, cyclobutyl, cyclopentyl, cyclohexyl, etc.; polycyclic cycloalkyl includes spiro, fused and bridged cycloalkyl.

The term "heterocycloalkyl" means a monovalent saturated or partially unsaturated monocyclic or bicyclic ring system of 3 to 8 ring atoms, containing 1, 2, or 3 ring heteroatoms selected from N, O and S, with the remaining ring atoms being carbon. In particular embodiments, heterocycloalkyl is a monovalent saturated monocyclic ring system of 4 to 7 ring atoms, containing 1, 2, or 3 ring heteroatoms selected from N, O and S, the remaining ring atoms being carbon. Examples of monocyclic saturated heterocycloalkyl are aziridinyl, oxiranyl, azetidinyl, oxetanyl, pyrrolidinyl, tetrahydrofuranyl, tetrahydro-thienyl, pyrazolidinyl, imidazolidinyl, oxazolidinyl, isoxazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, oxazepanyl and thiazinanyl. Examples of bicyclic saturated heterocycloalkyl are 8-aza-bicyclo[3.2.1]octyl, quinuclidinyl, 8-oxa-3-aza-bicyclo[3.2.1]octyl, 9-aza-bicyclo[3.3.1]nonyl, 3-oxa-9-aza-bicyclo[3.3.1]nonyl, 3-thia-9-aza-bicyclo[3.3.1]nonyl and 2,6-diaza-spiro[3.3]heptanyl. Examples of partially unsaturated heterocycloalkyl are dihydrofuranyl, imidazolinyl, dihydro-oxazolyl, tetrahydropyridinyl, or dihydropyranyl. More particular examples of heterocycloalkyl are pyrrolidinyl, pyrazolidinyl, imidazolidinyl, oxazolidinyl, isoxazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, oxazepanyl, thiazinanyl and 2,6-diaza-spiro[3.3]heptanyl. Even more particular examples of heterocycloalkyl are pyrrolidinyl, piperidinyl, thiomorpholinyl, thiazinanyl and 2,6-diaza-spiro[3.3]heptanyl.

The term "halocycloalkyl" means a cycloalkyl group in which at least one of the hydrogen atoms of the cycloalkyl group has been replaced by a same or different halogen atom, particularly fluorine atom. Examples of halocycloalkyl include fluorocyclopropyl, difluorocyclopropyl, fluorocyclobutyl and difluorocyclobutyl.

The term "C₁-C₆ alkylsulfonyl" refers to a C₁-C₆ alkyl group in which one H atom is substituted by a sulfonyl group, for example, methylsulfonyl, ethylsulfonyl, etc.

The term "C₁-C₆ alkylacyl" refers to a C₁-C₆ alkyl group in which one H atom is substituted by an acyl group, for example, formyl, acetyl, etc.

The term "C₁-C₆ alkylamido" refers to a C₁-C₆ alkyl group in which one H atom is substituted by an amido group, for example, carboxamido, acetamido, etc.

The term "aryl" or "aromatic ring" refers to a 6 to 12 membered all-carbon monocyclic or fused polycyclic (i.e., rings sharing adjacent pairs of carbon atoms) group having a conjugated π--electron system, preferably a 6 to 12 membered all-carbon monocyclic or fused polycyclic (i.e., rings sharing adjacent pairs of carbon atoms) group having a conjugated π-electron system, more preferably 8 to 10 membered, most preferably 6 to 8 membered, such as phenyl and naphthyl.

The term "heteroaryl" or "heteroaromatic ring" refers to a heteroaromatic system containing 1 to 3 heteroatoms and 5 to 10 ring atoms, wherein heteroatoms are selected from oxygen, sulfur, and nitrogen. Heteroaryl is preferably a 5- to 8-membered ring, more preferably a 5- or 6-membered ring, such as pyrrolyl, furanyl, thienyl, imidazolyl, oxazolyl, thiazolyl, triazolyl, oxadiazolyl, thiadiazolyl, tetrazolyl, pyridyl, pyrazinyl, pyrazolyl, pyridazinyl, pyrimidinyl, triazinyl, azepinyl, diazepinyl, isoxazolyl, benzofuranyl, isothiazolyl, benzothienyl, indolyl, isoindolyl, isobenzofuranyl, benzimidazolyl, benzoxazolyl, benzoisoxazolyl, benzothiazolyl, benzoisothiazolyl, benzoxadiazolyl, benzothiadiazolyl, benzotriazolyl, purinyl, quinolinyl, isoquinolinyl, quinazolinyl and quinoxalinyl. Particularly, the heteroaryl include pyrrolyl, furanyl, thienyl, imidazolyl, oxazolyl, thiazolyl, triazolyl, oxadiazolyl, thiadiazolyl, tetrazolyl, pyridyl, pyrazinyl, pyrazolyl, pyridazinyl, pyrimidinyl, isoxazolyl and isothiazolyl. More particularly, the heteroaryl include imidazolyl, oxazolyl, thiazolyl, oxadiazolyl, thiadiazolyl, tetrazolyl, pyridyl, pyrazinyl, pyrazolyl, pyridazinyl, pyrimidinyl, isoxazolyl and isothiazolyl.

The term "fused heteroaryl" refers to a fused bicyclic ring system containing 1 to 4 heteroatoms selected from N, O or S and their oxidized states and possessing aromaticity, which may be a fusion of heteroaryl group and an aryl group, or a fusion of two heteroaryls, wherein either heteroaryl or aryl moiety can serve as the attachment point. Non-limiting examples include benzothienyl, benzofuranyl, indolyl, isoindolyl, benzoxazolyl, benzimidazolyl, benzothiazolyl, benzoisoxazolyl, isobenzofuranyl, benzothiazolyl, benzoisothiazolyl, benzoxadiazolyl, benzothiadiazolyl, benzotriazolyl, purinyl, indazolyl, quinolinyl, isoquinolinyl, quinazolinyl, quinoxalinyl, benzopyridazinyl.

The term "cycloalkyloxy" or "cycloalkoxy" refers to cycloalkyl-O-, wherein cycloalkyl is as defined above.

The term "heterocyclyloxy or "heterocycloalkoxy" refers to heterocyclyl-O-, wherein heterocyclyl is as defined above.

The term "aryloxy" refers to aryl-O-, wherein aryl is as defined above.

The term "heteroaryloxy" refers to heteroaryl-O-, wherein heteroaryl is as defined above.

The atoms of the compound molecules of the present invention may contain isotopes. Isotopic derivatization can generally achieve effects such as extending half-life, reducing clearance rate, improving metabolic stability, and enhancing in vivo activity. Additionally, an embodiment is included wherein at least one atom is replaced by an atom having the same atomic number (number of protons) but a different mass number (sum of protons and neutrons). Examples of isotopes included in the compounds of the present invention include hydrogen atoms, carbon atoms, nitrogen atoms, oxygen atoms, phosphorus atoms, sulfur atoms, fluorine atoms, chlorine atoms, which respectively include ²H, ³H, ¹³C, ¹⁴C, ¹⁵N, ¹⁷O, ¹⁸O, ³¹P, ³²P, ³⁵S, ¹⁸F, ³⁶Cl. In particular, radioactive isotopes that emit radiation upon their decay, such as ³H or ¹⁴C, can be used in pharmaceutical formulations or for local anatomical studies of compounds in vivo. Stable isotopes which neither decay nor vary in quantity, are non-radioactive, so they can be used safely. When atoms constituting the compound molecules of the present invention are isotopes, isotopes can be transformed according to general methods by replacing the reagents used in the synthesis with reagents containing the corresponding isotope.

The compounds of the present invention may contain non-natural proportions of isotopes at one or more of the atoms that constitute such compounds. For example, the compounds may be radiolabeled with radioactive isotopes, such as deuterium (²H), iodine-125 (¹²⁵I) or C-14 (¹⁴C). All isotopic variations of the compounds of the present invention, whether radioactive or not, are intended to be encompassed within the scope of the present invention.

Furthermore, one or more hydrogen atoms of the compound of the present invention are substituted by the isotope deuterium (²H). After deuteration, the compound of the present invention has effects such as extanding half-life, reducing clearance rate, improving metabolic stability, and enhancing in vivo activity.

The preparation methods for the isotopic derivatives generally include phase transfer catalysis. For example, a preferred deuteration method employs a phase transfer catalyst (e.g., tetraalkylammonium salt, NBu₄HSO₄). Using a phase transfer catalyst to exchange the methylene protons of diphenylmethane compounds results in higher deuterium incorporation compared to by reduction with deuterated silanes (e.g., triethylsilyl deuteride) in the presence of an acid (e.g., methanesulfonic acid), or reduction with sodium borodeuteride in the presence of a Lewis acid such as aluminum trichloride.

The term "pharmaceutically acceptable carrier" refers to any formulation carrier or medium that can deliver an effective amount of the active substance of the present invention, without interfering with the biological activity of the active substance, and being non-toxic and free of side effects to the host or patient. Representative carriers include water, oils of vegetable or mineral substnances, cream bases, lotion bases, ointment bases, etc. These bases include suspending agents, viscosity enhancers, penetration enhancers, etc. Their formulations are well known to those skilled in the art of cosmetics or topical pharmaceuticals. For additional information on carriers, reference may be made to Remington: The Science and Practice of Pharmacy, 21st Ed., Lippincott, Williams & Wilkins (2005), the contents of which are incorporated herein by reference.

The term "excipient" generally refers to the carriers, diluents and/or media required to formulate an effective pharmaceutical composition.

The term "effective amount" or "therapeutically effective amount" for a drug or pharmacologically active agent refers to an amount of the drug or agent that is non-toxic yet sufficient to achieve the desired effect. For the oral dosage forms of the present invention, an "effective amount" of one active substance in the composition refers to the amount required to achieve the desired effect when used in combination with another active substance in the composition. The determination of an effective amount varies from person to person and depends on the age and general condition of the recipient, as well as the specific active substance. The appropriate effective amount in an individual case can be determined by those skilled in the art according to routine experimentation.

The terms "active ingredient", "therapeutic agent", "active substance" or "active agent" refer to a chemical entity that is effective in treating a target disorder, disease or condition.

"Optional" or "optionally" means that the subsequently described event or circumstance may but is not required to occur, and the description includes instances where the event or circumstance occurs and instances where it does not.

### SPECIFIC MODE FOR CARRYING OUT THE INVENTION

The present invention will be described in further detail below in conjunction with examples, but the content of the invention is not limited to the examples.

### Example 1

### Synthesis of (R)-N-(4-(quinolin-6-yl)-5,6,7,8-tetrahydroisoquinolin-8-yl)propionamide

Specific synthetic scheme is as follows:

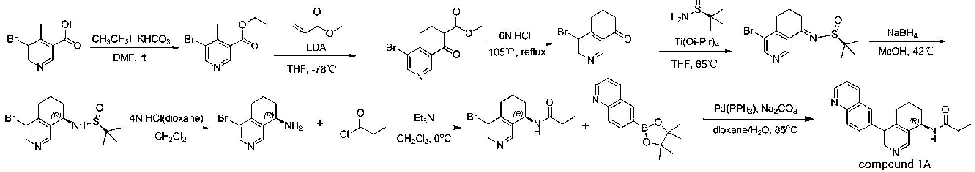

### Step A: Synthesis of ethyl 5-bromo-4-methylnicotinate

5-Bromo-4-methylnicotinic acid (50.0 g, 231.45 mmol) and ethyl iodide (39.7 g, 254.59 mmol) were dissolved in 500 mL of N,N-dimethylformamide, to which was added potassium bicarbonate (46.3 g, 462.90 mmol). The mixed solution was degassed and under a nitrogen atmosphere, and then stirred at room temperature for 12 hours.

After the reaction was completed, the mixture was filtered. To the filtrate was added water, and the mixture was extracted with ethyl acetate (300 mL×3). The organic phases were combined, washed with saturated brine (500 mL), then dried over anhydrous sodium sulfate, and finally concentrated under reduced pressure. The obtained residue was purified by silica gel column chromatography (eluent: ethyl acetate/n-hexane = 1/10) to yield 54.6 g of ethyl 5-bromo-4-methylnicotinate. [M+H]⁺ = 244.05.

### Step B: Synthesis of methyl 4-bromo-8-oxo-5,6,7,8-tetrahydroisoquinolin-7-carboxylate

At -78°C, to a solution of ethyl 5-bromo-4-methylnicotinate (54.6 g, 223.69 mmol) in tetrahydrofuran (500 mL) was added LDA (123 mL, 246.06 mmol, 2 M) dropwise. The mixture was stirred for 30 minutes, then a solution of methyl acrylate (48.1 g, 559.22 mmol) in tetrahydrofuran (200 mL) was added dropwise, and the mixture was stirred at -78°C for another 2 hours.

After the reaction was completed, 400 mL of 10% aqueous acetic acid solution was added to quench the reaction. The organic solvent was removed by rotary evaporation. The mixture was extracted with ethyl acetate (300 mL×3), and then the organic phases were combined, washed with saturated brine (500 mL), dried over anhydrous sodium sulfate, and finally concentrated under reduced pressure. The obtained residue was purified by silica gel column chromatography (eluent: ethyl acetate/n-hexane = 1/10) to yield 31.5 g of methyl 4-bromo-8-oxo-5,6,7,8-tetrahydroisoquinoline-7-carboxylate. [M+H]⁺ = 284.06.

### Step C: Synthesis of 4-bromo-6,7-dihydroisoquinolin-8(5H)-one

Methyl 4-bromo-8-oxo-5,6,7,8-tetrahydroisoquinoline-7-carboxylate (31.5 g, 110.87 mmol) was dissolved in 300 mL of aqueous hydrochloric acid (6 M), and the mixture was heated to 105°C and stirred under reflux for 16 hours.

After the reaction was completed, the solvent was removed by rotary evaporation. To the residue was added 300 mL of water, and the mixture was adjusted to pH ~9 with 1N aqueous sodium hydroxide solution. The mixture was extracted with ethyl acetate (200 mL×3). The organic phases were combined, washed with saturated brine (300 mL), then dried over anhydrous sodium sulfate, and finally concentrated under reduced pressure. The obtained residue was purified by silica gel column chromatography (eluent: ethyl acetate/n-hexane = 1/8) to yield 19.6 g of 4-bromo-6,7-dihydroisoquinolin-8(5H)-one. [M+H]⁺ = 226.05.

### Step D: Synthesis of (S)-N-(4-bromo-6,7-dihydroisoquinolin-8(5H)-ylidene)-2-methylpropane-2-sulfinamide

4-Bromo-6,7-dihydroisoquinolin-8(5H)-one (10.0 g, 44.23 mmol) was dissolved in 200 mL of tetrahydrofuran, to which were added (S)-tert-butanesulfinamide (5.9 g, 48.66 mmol) and titanium tetraisopropoxide (37.7 g, 132.70 mmol), and the mixture was heated to 65°C under nitrogen protection and stirred for 24 hours.

After the reaction was completed, 100 mL of water was added to quench the reaction. The solid was removed by filtration, and the filtrate was concentrated. Water (100 mL) was added to the residue, and the mixture was extracted with ethyl acetate (100 mL×3). The combined organic phases were washed with saturated brine (100 mL), dried over anhydrous sodium sulfate, and concentrated under reduced pressure. The obtained residue was purified by silica gel column chromatography (eluent: ethyl acetate/n-hexane = 1/5) to yield 13.3 g of (S)-N-(4-bromo-6,7-dihydroisoquinolin-8(5H)-ylidene)-2-methylpropane-2-sulfinamide. [M+H]⁺ = 329.12.

### Step E: Synthesis of (S)-N-((R)-4-bromo-5,6,7,8-tetrahydroisoquinolin-8-yl)-2-methylpropane-2-sulfinamide

At -42°C, sodium borohydride (2.3 g, 60.59 mmol) was added in portions to a methanol (400 mL) solution of (S)-N-(4-bromo-6,7-dihydroisoquinolin-8(5H)-ylidene)-2-methylpropane-2-sulfinamide (13.3 g, 40.39 mmol), and the mixture was stirred at -42°C for another 1 hour.

After the reaction was completed, 100 mL of water was added to quench the reaction. The solvent was removed by rotary evaporation. To the residue was added 100 mL of water, and the mixture was extracted with ethyl acetate (100 mL×3). The organic phases were combined, washed with saturated brine (100 mL), then dried over anhydrous sodium sulfate, and finally concentrated under reduced pressure. The obtained residue was purified by silica gel column chromatography (eluent: methanol/dichloromethane = 1/20) to obtain 11.1 g of (S)-N-((R)-4-bromo-5,6,7,8-tetrahydroisoquinolin-8-yl)-2-methylpropane-2-sulfinamide. [M+H]⁺ = 331.06. ¹H NMR (400 MHz, CDCl₃) δ 8.58 (s, 1H), 8.57 (s, 1H), 4.59--4.51 (m, 1H), 3.41 (d, J = 10.0 Hz, 1H), 2.83-2.68 (m, 2H), 2.38--2.28 (m, 1H), 2.05--1.95 (m, 2H), 1.94--1.84 (m, 1H), 1.29 (s, 9H).

### Step F: Synthesis of (R)-4-bromo-5,6,7,8-tetrahydroisoquinolin-8-amine

To a solution of (S)-N-((R)-4-bromo-5,6,7,8-tetrahydroisoquinolin-8-yl)-2-methylpropane-2-sulfinamide (11.1 g, 9.86 mmol) in dichloromethane (100 mL) was added 40 mL of hydrogen chloride-dioxane solution (4 M), and the reaction was stirred at room temperature for 5 hours.

After the reaction was completed, the mixture was concentrated by rotary evaporation. To the residue was added 100 mL of water, and the mixture was adjusted to pH 9 with sodium hydroxide solution (1 M). The mixture was extracted with ethyl acetate (100 mL×3). The organic phases were combined, washed with saturated brine (100 mL), then dried over anhydrous sodium sulfate, and finally concentrated under reduced pressure. The obtained residue was purified by silica gel column chromatography (eluent: methanol/dichloromethane = 1/10) to yield 7.2 g of (R)-4-bromo-5,6,7,8-tetrahydroisoquinolin-8-amine. [M+H]⁺ = 227.11.

### Step G: Synthesis of (R)-N-(4-bromo-5,6,7,8-tetrahydroisoquinolin-8-yl)propionamide

(R)-4-Bromo-5,6,7,8-tetrahydroisoquinolin-8-amine (7.2 g, 31.70 mmol) and triethylamine (8.8 mL, 63.41 mmol) were dissolved in dichloromethane (100 mL), to which was added propionyl chloride (3.1 mL, 34.87 mmol) dropwise at 0°C. The mixture was stirred at room temperature for 5 minutes.

After the reaction was completed, water was added to the mixture, and the mixture was extracted with dichloromethane (100 mL×3). The organic phases were combined, washed with saturated brine (100 mL), then dried over anhydrous sodium sulfate, and finally concentrated under reduced pressure. The obtained residue was purified by silica gel column chromatography (eluent: methanol/dichloromethane = 1/20) to yield 8.5 g of (R)-N-(4-bromo-5,6,7,8-tetrahydroisoquinolin-8-yl)propionamide. [M+H]⁺ = 283.12.

### Step H: Synthesis of (R)-N-(4-(quinolin-6-yl)-5,6,7,8-tetrahydroisoquinolin-8-yl)propionamide

(R)-4-Bromo-5,6,7,8-tetrahydroisoquinolin-8-amine (100 mg, 0.35 mmol) and 6-quinolineboronic acid pinacol ester (108 mg, 0.42 mmol) were dissolved in a mixed solvent of 5.0 mL dioxane and 1.0 mL water, to which were added sodium carbonate (76 g, 0.71 mmol) and tetrakis(triphenylphosphine)palladium (8 mg, 0.0071 mmol). The mixture was under a nitrogen atmosphere and reacted at 85°C for 6 hours.

After the reaction was completed, the obtained suspension was filtered. The filter cake was washed with dichloromethane, and the filtrate was concentrated under reduced pressure. The obtained residue was purified by silica gel column chromatography (eluent: methanol/dichloromethane = 1/20) to yield 87 mg of (R)-N-(4-(quinolin-6-yl)-5,6,7,8-tetrahydroisoquinolin-8-yl)propionamide. [M+H]⁺ = 332.09. ¹H NMR (400 MHz, DMSO-d6) δ 9.11 (dd, J = 4.5, 1.7 Hz, 1H), 8.69-8.61 (m, 3H), 8.46 (d, J = 8.0 Hz, 1H), 8.25 (d, J = 8.7 Hz, 1H), 8.19 (d, J = 2.0 Hz, 1H), 7.94 (dd, J = 8.7, 2.0 Hz, 1H), 7.78 (dd, J = 8.3, 4.5 Hz, 1H), 5.18 (q, J = 6.8 Hz, 1H), 2.87-2.73 (m, 2H), 2.27-2.16 (m, 2H), 2.01-1.91 (m, 1H), 1.89-1.72 (m, 3H), 1.08 (t, J = 7.6 Hz, 3H).

### Example 2-7

| | Chemical Name | Structure | Preparation and characterization |
|---|---|---|---|
| **Example 2** | **(*R*)-*N*-(5,6,7,8-tetrahydro-[4,6'-biisoquinolin]-8-yl)propionamide** | 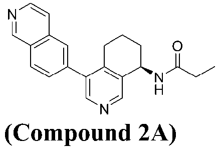 | (*R*)-*N*-(5,6,7,8-tetrahydro-[4,6'-biisoquinolin]-8-yl)propionamide was obtained following the specific experimental procedures described in Example 1. [M+H]⁺ = 332.07. |
| **Example 3** | **(*R*)-*N*-(4-(quinolin-7-yl)-5,6,7,8-tetrahydroisoquinolin-8-yl)propionamide** | 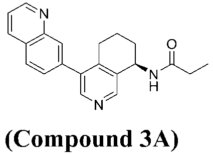 | (*R*)-*N*-(4-(quinolin-7-yl)-5,6,7,8-tetrahydroisoquinolin-8-yl)propionamide was obtained following the specific experimental procedures described in Example 1. [M+H]⁺ = 332.07. NMR data: ¹H NMR (400 MHz, DMSO-*d*₆) δ 8.98 (qd, *J* = 3.0, 1.3 Hz, 1H), 8.47 (d, *J =* 8.2 Hz, 1H), 8.42 (d, *J =* 2.4 Hz, 1H), 8.37 (d, *J* = 2.3 Hz, 1H), 8.34 (d, *J =* 8.6 Hz, 1H), 8.11 (d, *J =* 8.5 Hz, 1H), 7.98 (s, 1H), 7.66 - 7.59 (m, 2H), 5.18-5.13 (m, 1H), 2.66 (q, *J* = 5.2 Hz, 2H), 2.24-2.13 (m, 2H), 1.99-1.66 (m, 4H), 1.08 (t, *J* = 7.6 Hz, 3H). |
| **Example 4** | **(*R*)-*N*-(5,6,7,8-tetrahydro-[4,7'-biisoquinolin]-8-yl)propionamide** | 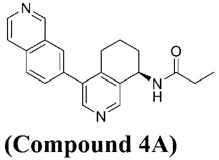 | (*R*)-*N*-(5,6,7,8-tetrahydro-[4,7'-biisoquinolin]-8-yl)propionamide was obtained following the specific experimental procedures described in Example 1. [M+H]⁺ = 332.07. |
| **Example 5** | **(*R*)-*N*-(4-(2-methylquinolin-6-yl)-5,6,7,8-tetrahydroisoquinolin-8-yl)propionamide** | 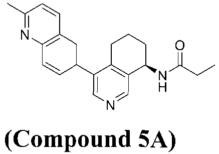 | (*R*)-*N*-(4-(2-methylquinolin-6-yl)-5,6,7,8-tetrahydroisoquinolin-8-yl)propionamide was obtained following the specific experimental procedures described in Example 1. [M+H]⁺ = 346.06. |
| **Example 6** | **(*R*)*-N*-(4-(8-chloroquinolin-6-yl)-5,6,7,8-tetrahydroisoquinolin-8-yl)propionamide** | 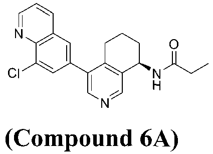 | (*R*)-*N*-(4-(8-chloroquinolin-6-yl)-5,6,7,8-tetrahydroisoquinolin-8-yl)propionamide was obtained following the specific experimental procedures described in Example 1. [M+H]⁺ = 366.12. |
| **Example 7** | **(*R*)-*N*-(4-(4-methyl)-5,6,7,8-tetrahydroisoquinolin-8-yl)propionamide** | 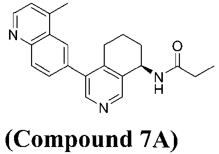 | (*R*)-*N*-(4-(4-methylquinolin-6-yl)-5,6,7,8-tetrahydroisoquinolin-8-yl)propionamide was obtained following the specific experimental procedures described in Example 1. [M+H]⁺ = 346.06. NMR data: ¹H NMR (400 MHz, DMSO-*d*₆) δ 8.82 (d, *J* = 4.3 Hz, 1H), 8.42 (d, *J =* 0.7 Hz, 1H), 8.39 (s, 1H), 8.32 (d, *J* = 8.4 Hz, 1H), 8.11 (d, *J =* 8.6 Hz, 1H), 8.03 (d, *J =* 1.9 Hz, 1H), 7.78 (dd, *J =* 8.6, 1.9 Hz, 1H), 7.46 (dd,*J* = 4.4, 1.0 Hz, 1H), 5.15 (q, *J* = 6.5 Hz, 1H), 2.73 (d, *J =* 1.0 Hz, 3H), 2.67 (t, *J =* 6.1 Hz, 2H), 2.24-2.14 (m, 2H), 1.97-1.66 (m, 4H), 1.09 (t, *J =* 7.6 Hz, 3H). |

### Example 8

### Synthesis of (R)-N-(4-(2-cyanoquinolin-6-yl)-5,6,7,8-tetrahydroisoquinolin-8-yl)propionamide

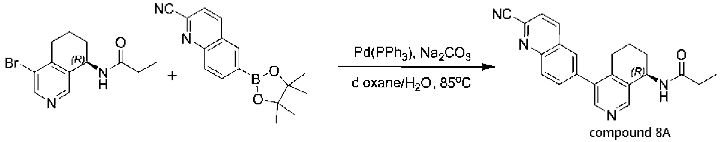

(*R*)*-N*-(4-(2-cyanoquinolin-6-yl)-5,6,7,8-tetrahydroisoquinolin-8-yl)propionamide was obtained following the specific experimental procedures described in Example 1.

### Specific operation steps:

### Step A:

(*R*)-4-bromo-5,6,7,8-tetrahydroisoquinolin-8-amine was obtained following the method of Example 1, [M+H]⁺ = 283.12

### Step B:

(R)-4-Bromo-5,6,7,8-tetrahydroisoquinolin-8-amine (100 mg, 0.35 mmol) and 2-cyanoquinolin-6-ylboronic acid pinacol ester (119 mg, 0.42 mmol) were dissolved in a mixed solvent of 5.0 mL dioxane and 1.0 mL water, to which were added sodium carbonate (76 g, 0.71 mmol) and tetrakis(triphenylphosphine)palladium (8 mg, 0.0071 mmol). The mixture was under a nitrogen atmosphere and reacted at 85°C for 6 hours.

After the reaction was completed, the obtained suspension was filtered, and the filter cake was washed with dichloromethane. The filtrate was concentrated under reduced pressure, and the obtained residue was purified by silica gel column chromatography (eluent: methanol/dichloromethane = 1/20) to yield 109 mg of (R)-N-(4-(2-cyanoquinolin-6-yl)-5,6,7,8-tetrahydroisoquinolin-8-yl)propionamide.

[M+H]⁺ =357.00. NMR data: ¹H NMR (400 MHz, DMSO-*d*₆) δ 8.74 (d, *J* = 8.5 Hz, 1H), 8.45 (s, 1H), 8.39 (s, 1H), 8.34 (d, *J* = 8.4 Hz, 1H), 8.25 (d, *J* = 8.7 Hz, 1H), 8.18 (d, *J* = 2.0 Hz, 1H), 8.14 (d, *J* = 8.4 Hz, 1H), 7.99 (dd, *J* = 8.7, 2.0 Hz, 1H), 5.15 (q, *J* = 6.5 Hz, 1H), 2.71-2.63 (m, 2H), 2.26-2.12 (m, 2H), 1.99-1.66 (m, 4H), 1.09 (t, *J =* 7.6 Hz, 3H).

### Example 9-25

| | Chemical Name | Structure | Preparation and characterization |
|---|---|---|---|
| **Example 9** | **(*R*)*-N*-(4-(7-fluoroquinolin-6-yl)-5,6,7,8-tetrahydroisoquinolin-8-yl)propionamide** | 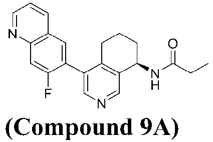 | (*R*)*-N*-(4-(7-fluoroquinolin-6-yl)-5,6,7,8-tetrahydroisoquinolin-8-yl)propionamide was obtained following the specific experimental procedures described in Example 1. [M+H]⁺ =350.01. NMR data: ¹H NMR (400 MHz, DMSO-*d*₆) δ 9.00 (dd, *J =* 4.2, 1.7 Hz, 1H), 8.48 (d, *J =* 10.8 Hz, 2H), 8.37 (s, 2H), 8.07 (d, *J* = 8.1 Hz, 1H), 7.94 (d, *J =* 11.2 Hz, 1H), 7.62 (dd, *J =* 8.3, 4.3 Hz, 1H), 5.15 (q, *J* = 6.7 Hz, 1H), 2.71-2.63 (m, 2H), 2.27 - 2.12 (m, 2H), 1.97-1.65 (m, 4H), 1.09 (t, *J* = 7.6 Hz, 3H). |
| **Example 10** | **(*R*)-*N*-(4-(2-methoxyquinolin-6-yl)-5,6,7,8-tetrahydroisoquinolin-8-yl)propionamide** | 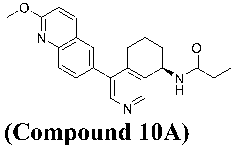 | (*R*)-*N-*(4-(2-methoxyquinolin-6-yl)-5,6,7,8-tetrahydroisoquinolin-8-yl)propionamide was obtained following the specific experimental procedures described in Example 1. [M+H]⁺ =362.05. |
| **Example 11** | **(*R*)-*N*-(4-(2-(trifluoromethyl)quinolin-6-yl)-5,6,7,8-tetrahydroisoquinolin-8-yl)propionamide** | 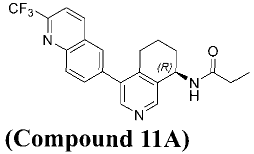 | (*R*)-*N*-(4-(2-(trifluoromethyl)quinolin-6-yl)-5,6,7,8-tetrahydroisoquinolin-8-yl)propionamide was obtained following the specific experimental procedures described in Example 1. [M+H]⁺ = 400.00. NMR data: ¹H NMR (400 MHz, DMSO-*d*₆) δ 8.80 (d, *J* = 8.6 Hz, 1H), 8.45 (s, 1H), 8.39 (s, 1H), 8.34 (d, *J* = 8.4 Hz, 1H), 8.28 (d, *J* = 8.7 Hz, 1H), 8.20 (d, *J* = 2.0 Hz, 1H), 8.08 (d, *J* = 8.5 Hz, 1H), 7.97 (dd, *J =* 8.7, 2.0 Hz, 1H), 5.16 (q, *J* = 6.6 Hz, 1H), 2.71-2.61 (m, 2H), 2.27-2.12 (m, 2H), 1.98-1.66 (m, 4H), 1.09 (t, *J* = 7.6 Hz, 3H). |
| **Example 12** | **(*R*)-*N*-(4-(7-methylquinolin-6-yl)-5,6,7,8-tetrahydroisoquinolin-8-yl)propionamide** | 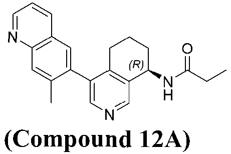 | (*R*)-*N*-(4-(7-methylquinolin-6-yl)-5,6,7,8-tetrahydroisoquinolin-8-yl)propionamide was obtained following the specific experimental procedures described in Example 1. [M+H]⁺ =346.09. NMR data: ¹H NMR (400 MHz, DMSO-*d*₆) δ 8.92 (dt, *J =* 4.0, 1.9 Hz, 1H), 8.42 (s, 1H), 8.40-8.29 (m, 2H), 8.23 (d, *J* = 1.7 Hz, 1H), 8.01 (s, 1H), 7.74 (d, *J =* 35.3 Hz, 1H), 7.55-7.51 (m, 1H), 5.17-5.10 (m, 1H), 2.47-2.26 (m, 2H), 2.33-2.13 (m, 5H), 1.96-1.63 (m, 4H), 1.09 (t, *J* = 7.6 Hz, 3H). |
| **Example 13** | **(*R*)-*N*-(4-(7-methoxyquinolin-6-yl)-5,6,7,8-tetrahydroisoquinolin-8-yl)propionamide** | 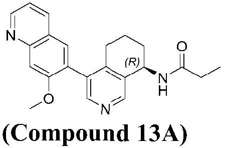 | (*R*)-*N*-(4-(7-methoxyquinolin-6-yl)-5,6,7,8-tetrahydroisoquinolin-8-yl)propionamide was obtained following the specific experimental procedures described in Example 1. [M+H]⁺ =362.09. NMR data: ¹H NMR (400 MHz, DMSO-*d*₆) δ 8.91-8.86 (m, *J* = 4.2, 1.9 Hz, 1H), 8.46-8.27 (m, 3H), 8.25 (s, 1H), 7.78 (d, *J* = 32.2 Hz, 1H), 7.55 (s, 1H), 7.47-7.36 (m, 1H), 5.13 (d, *J* = 7.0 Hz, 1H), 3.91 (s, 3H), 2.49-2.30 (m, 2H), 2.27-2.13 (m, 2H), 1.96-1.58 (m, 4H), 1.09 (q, *J* = 7.6 Hz, 3H). |
| **Example 14** | **(*R*)-*N*-(4-(8-fluoroquinolin-6-yl)-5,6,7,8-tetrahydroisoquinolin-8-yl)propionamide** | 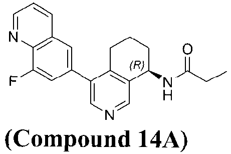 | (*R*)-*N*-(4-(8-fluoroquinolin-6-yl)-5,6,7,8-tetrahydroisoquinolin-8-yl)propionamide was obtained following the specific experimental procedures described in Example 1. [M+H]⁺ =350.08. NMR data: ¹H NMR (400 MHz, DMSO-*d*₆) δ 9.03 (dd, *J =* 4.2, 1.6 Hz, 1H), 8.54-8.51 (m, 1H), 8.43 (s, 1H), 8.38 (s, 1H), 8.33 (d, *J* = 8.4 Hz, 1H), 7.86 (d, *J* = 1.8 Hz, 1H), 7.74-7.68 (m, 2H), 5.18-5.10 (m, 1H), 2.69 (t, *J* = 5.9 Hz, 2H), 2.26-2.12 (m, 2H), 1.97-1.68 (m, 4H), 1.08 (t, *J* = 7.6 Hz, 3H). |
| **Example 15** | **(*R*)-*N*-(4-(8-methylquinolin-6-yl)-5,6,7,8-tetrahydroisoquinolin-8-yl)propionamide** | 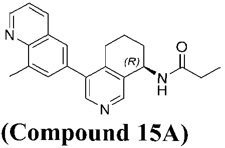 | (*R*)-*N*-(4-(8-methylquinolin-6-yl)-5,6,7,8-tetrahydroisoquinolin-8-yl)propionamide was obtained following the specific experimental procedures described in Example 1. [M+H]⁺ = 346.05. NMR data: ¹H NMR (400 MHz, DMSO-*d*₆) δ 8.99 (dd, *J =* 4.2, 1.8 Hz, 1H), 8.43-8.38 (m, 2H), 8.36-8.31 (m, 2H), 7.82 (d, *J =* 2.0 Hz, 1H), 7.65 (dd, *J =* 2.1, 1.1 Hz, 1H), 7.62 (dd, *J* = 8.3, 4.2 Hz, 1H), 5.14 (q, *J* = 6.5 Hz, 1H), 2.79 (s, 3H), 2.69-2.65 (m, 2H), 2.26-2.12 (m, 2H), 1.98-1.65 (m, 4H), 1.09 (t, *J* = 7.6 Hz, 3H). |
| **Example 16** | **(*R*)-*N*-(4-(cinnolin-6-yl)-5,6,7,8-tetrahydroisoquinolin-8-yl)propionamide** | 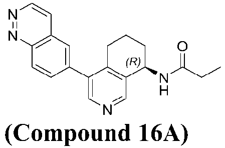 | (*R*)-*N*-(4-(cinnolin-6-yl)-5,6,7,8-tetrahydroisoquinolin-8-yl)propionamide was obtained following the specific experimental procedures described in Example 1. [M+H]⁺ = 333.07. |
| **Example 17** | **(*R*)-*N*-(4-(quinoxalin-6-yl)-5,6,7,8-tetrahydroisoquinolin-8-yl)propionamide** | 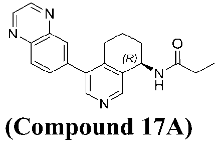 | (*R*)-*N*-(4-(quinoxalin-6-yl)-5,6,7,8-tetrahydroisoquinolin-8-yl)propionamide was obtained following the specific experimental procedures described in Example 1. [M+H]⁺ = 333.04. |
| **Example 18** | **(*R*)-*N*-(4-(quinazolin-6-yl)-5,6,7,8-tetrahydroisoquinolin-8-yl)propionamide** | 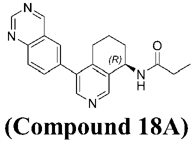 | (*R*)-*N*-(4-(quinazolin-6-yl)-5,6,7,8-tetrahydroisoquinolin-8-yl)propionamide was obtained following the specific experimental procedures described in Example 1. [M+H]⁺ = 333.04. |
| **Example 19** | **(*R*)-*N*-(4-(3-methylbenzo[*d*]isoxazol-5-yl)-5,6,7,8-tetrahydroisoquinolin-8-yl)propionamide** | 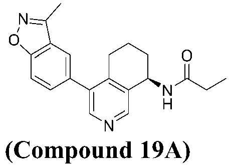 | (*R*)-*N*-(4-(3-methylbenzo[*d*]isoxazol-5-yl)-5,6,7,8-tetrahydroisoquinolin-8-yl)propionamide was obtained following the specific experimental procedures described in Example 1. [M+H]⁺ = 336.05. |
| **Example 20** | **(*R*)-*N*-(4-(1-methyl-1*H-*indazol-6-yl)-5,6,7,8-tetrahydroisoquinolin-8-yl)propionamide** | 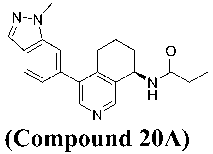 | (*R*)-*N*-(4-(1-methyl-1*H-*indazol-6-yl)-5,6,7,8-tetrahydroisoquinolin-8-yl)propionamide was obtained following the specific experimental procedures described in Example 1. [M+H]⁺ = 335.09. |
| **Example 21** | **(*R*)-*N*-(4-(1-methyl-1*H-*indazol-5-yl)-5,6,7,8-tetrahydroisoquinolin-8-yl)propionamide** | 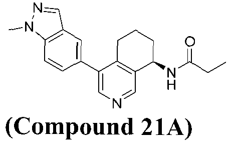 | (*R*)-*N*-(4-(1-methyl-1*H-*indazol-5-yl)-5,6,7,8-tetrahydroisoquinolin-8-yl)propionamide was obtained following the specific experimental procedures described in Example 1. [M+H]⁺ = 335.06. |
| **Example 22** | **(*R*)-*N*-(4-(1-methyl-1*H-*indol-5-yl)-5,6,7,8-tetrahydroisoquinolin-8-yl)propionamide** | 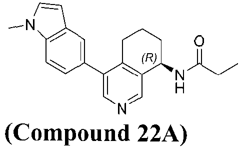 | (*R*)-*N*-(4-(1-methyl-1*H-*indol-5-yl)-5,6,7,8-tetrahydroisoquinolin-8-yl)propionamide was obtained following the specific experimental procedures described in Example 1. [M+H]⁺ = 334.12. |
| **Example 23** | **(*R*)-*N*-(4-(imidazo[1,2-a]pyridin-6-yl)-5,6,7,8-tetrahydroisoquinolin-8-yl)propionamide** | 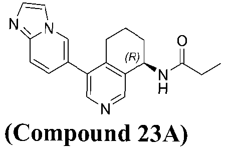 | (*R*)-*N*-(4-(imidazo[1,2-a]pyridin-6-yl)-5,6,7,8-tetrahydroisoquinolin-8-yl)propionamide was obtained following the specific experimental procedures described in Example 1. [M+H]⁺ = 321.06. |
| **Example 24** | **(*R*)-*N*-(4-(benzoxazol-6-yl)-5,6,7,8-tetrahydroisoquinolin-8-yl)propionamide** | 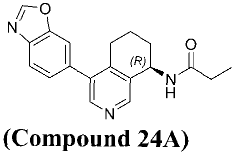 | (*R*)-*N*-(4-(benzoxazole-6-yl)-5,6,7,8-tetrahydroisoquinolin-8-yl)propionamide was obtained following the specific experimental procedures described in Example 1. [M+H]⁺ = 322.04. |
| **Example 25** | **(*R*)-*N*-(4-(benzothiazol-6-yl)-5,6,7,8-tetrahydroisoquinolin-8-yl)propionamide** | 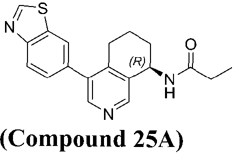 | (*R*)-*N*-(4-(benzothiazol-6-yl)-5,6,7,8-tetrahydroisoquinolin-8-yl)propionamide was obtained following the specific experimental procedures described in Example 1. [M+H]⁺ = 338.01. NMR data: ¹H NMR (400 MHz, DMSO-*d*₆) δ 9.48 (s, 1H), 8.41 (s, 1H), 8.35 (d, *J* = 7.5 Hz, 2H), 8.23-8.17 (m, 2H), 7.58-7.45 (m, 1H), 5.14 (q, *J* = 6.3 Hz, 1H), 2.64 (t, *J* = 6.0 Hz, 2H), 2.18 (qd, *J* = 7.4, 4.9 Hz, 2H), 1.97-1.65 (m, 4H), 1.08 (t, *J =* 7.6 Hz, 3H). |

### Example 26

### Synthesis of (R)-N-(1'-cyano-5,6,7,8-tetrahydro-[4,6'-biisoquinolin]-8-yl)propionamide

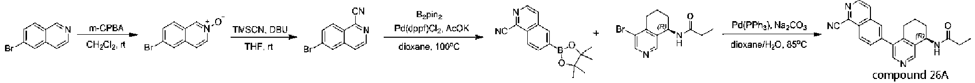

### Step A: Synthesis of 6-bromoquinoline 2-oxide

To a reaction flask were added 6-bromoisoquinoline (500 mg, 2.40 mmol), dichloromethane (10 mL), and m-chloroperoxybenzoic acid (621 mg, 3.60 mmol), and the mixture was allowed to react at room temperature for 15 hours.

After the reaction was completed, the mixture was adjusted to pH 9 with 1 mol/L aqueous sodium hydroxide solution, extracted with dichloromethane (20 mL×2), dried over sodium sulfate, and concentrated. The residue was slurried with ethyl acetate/n-hexane = 1/5 (10 mL) at room temperature for 1 hour and filtered to obtain 401 mg of 6-bromoquinoline-2-oxide as white solid , [M+H]⁺=224.04.

### Step B: Synthesis of 6-bromoisoquinolin-1-carbonitrile

To a reaction flask were added 6-bromoquinoline 2-oxide (400 mg, 1.79 mmol), anhydrous tetrahydrofuran (10 mL), followed by trimethylsilyl cyanide (354 mg, 3.57 mmol) and 1,8-diazabicyclo[5.4.0]undec-7-ene (326 mg, 2.14 mmol). The mixture was reacted at room temperature for 3 hours.

After the reaction was completed, tap water (10 mL) was added, the mixture was extracted with ethyl acetate (20 mL ×2), dried over sodium sulfate, and concentrated. The residue was purified by silica gel column chromatography (ethyl acetate/n-hexane = 1/3) to obtain 337 mg of 6-bromoisoquinoline-1-carbonitrile, [M+H]⁺ = 232.01.

### Step C: Synthesis of 6-(4,4,5,5-tetramethyl-1,3,2-dioxaborolan-2-yl)isoquinolin-1-carbonitrile

Dioxane (5 mL), 6-bromoisoquinoline-1-carbonitrile (300 mg, 1.29 mmol), bis(pinacolato)diboron (393 mg, 1.55 mmol), potassium acetate (317 mg, 3.23 mmol), 1,1'-bis(diphenylphosphino)ferrocene palladium(II) dichloride (47 mg, 0.07 mmol) were mixed, purged with nitrogen, heated to 100°C and allowed to react for 18 hours.

After the reaction was completed, the mixture was cooled to room temperature, diluted with water (10 mL), extracted with ethyl acetate (10 mL ×2),and concentrated. The residue was purified by silica gel column chromatography (eluent: ethyl acetate/n-hexane = 1/3) to yield 315 mg of 6-(4,4,5,5-tetramethyl-1,3,2-dioxaborolan-2-yl)isoquinoline-1-carbonitrile, as a yellow solid.

### Step D: Synthesis of (R)-N-(1'-cyano-5,6,7,8-tetrahydro-[4,6'-biisoquinolin]-8-yl)propionamide

Following a synthesis method similar to Step H in Example 1, (R)-4-bromo-5,6,7,8-tetrahydroisoquinolin-8-amine was coupled with 6-(4,4,5,5-tetramethyl-1,3,2-dioxaborolan-2-yl)isoquinoline-1-carbonitrile to obtain (*R*)-*N*-(1'-cyano-5,6,7,8-tetrahydro-[4,6'-biisoquinolin]-8-yl)propionamide. [M+H]⁺ = 357.00. NMR data: ¹H NMR (400 MHz, DMSO-*d*₆) δ 8.80 (d, *J* = 5.5 Hz, 1H), 8.46 (d, *J =* 0.7 Hz, 1H), 8.38 (s, 1H), 8.37-8.30 (m, 3H), 8.25 (d, *J =* 1.6 Hz, 1H), 7.99 (dd, *J =* 8.6, 1.7 Hz, 1H), 5.15 (q, *J* = 6.6 Hz, 1H), 2.65 (q, *J =* 5.6 Hz, 2H), 2.19 (qd, *J =* 7.4, 5.0 Hz, 2H), 1.94 (dd, *J* = 9.9, 4.8 Hz, 1H), 1.87-1.65 (m, 3H), 1.09 (t, *J =* 7.6 Hz, 3H).

### Example 27

### Synthesis of (R)-N-(1'-(methylsulfonyl)-5,6,7,8-tetrahydro-[4,6'-biisoquinolin]-8-yl)propionamide

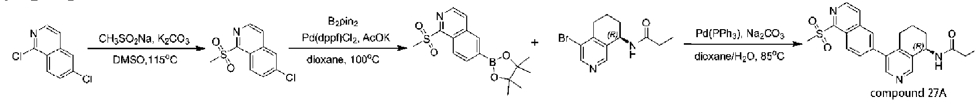

### Step A: 6-chloro-1-(methylsulfonyl)isoquinoline

To a reaction flask were added 1,6-dichloroisoquinoline (450 mg, 2.27 mmol), sodium methylsulfinate (348 mg, 3.41 mmol), potassium carbonate (784 mg, 5.67 mmol), and dimethyl sulfoxide (10 mL). The mixture was purged with nitrogen, heated to 115°C and allowed to react for 2 hours.

After the reaction was completed, the mixture was allowed to cool to room temperature. Water (30 mL) was slowly added to the reaction mixture, and the resulting mixture was stirred for 1 hour to promote crystallization. The precipitate was collected by filtration to obtain the crude product, which was further purified by silica gel column chromatography (ethyl acetate/n-hexane = 1/5) to obtain 150 mg of 6-chloro-1-(methylsulfonyl)isoquinoline as a white solid, [M+H]⁺ = 241.93.

### Step B: 1-(methylsulfonyl)-6-(4,4,5,5-tetramethyl-1,3,2-dioxaborolan-2-yl)isoquinoline

1-(methylsulfonyl)-6-(4,4,5,5-tetramethyl-1,3,2-dioxaborolan-2-yl)isoquinoline was obtained following the synthesis method referred to Step C in Example 26.

### Step C: Synthesis of (R)-N-(1'-(methylsulfonyl)-5,6,7,8-tetrahydro-[4,6'-biisoquinolin]-8-yl)propionamide

Following a synthesis method similar to Step H in Example 1 , (R)-4-bromo-5,6,7,8-tetrahydroisoquinolin-8-amine was coupled with 1-(methylsulfonyl)-6-(4,4,5,5-tetramethyl-1,3,2-dioxaborolan-2-yl)isoquinoline, to obtain (*R*)-*N*-(1'-(methylsulfonyl)-5,6,7,8-tetrahydro-[4,6'-biisoquinolin]-8-yl)propionamide. [M+H]⁺ = 410.00. NMR data: ¹H NMR (400 MHz, DMSO-*d*₆) δ 8.89 (d, *J* = 8.8 Hz, 1H), 8.68 (d, *J* = 5.6 Hz, 1H), 8.46 (s, 1H), 8.39 (s, 1H), 8.34 (d, *J* = 8.4 Hz, 1H), 8.29 (dd, *J* = 5.7, 0.9 Hz, 1H), 8.25 (d, *J* = 1.7 Hz, 1H), 7.94 (dd, *J =* 8.8, 1.8 Hz, 1H), 5.15 (d, *J* = 7.2 Hz, 1H), 3.62 (s, 3H), 2.70-2.63 (m, 2H), 2.19 (qd, *J* = 7.4, 5.1 Hz, 2H), 1.99-1.65 (m, 4H), 1.09 (t, *J* = 7.6 Hz, 3H).

### Example 28-33

| | Chemical Name | Structure | Preparation and characterization |
|---|---|---|---|
| **Example 28** | **(*R*)-*N*-(1'-methyl-5,6,7,8-tetrahydro-[4,6'-biisoquinolin]-8-yl)propionamide** | 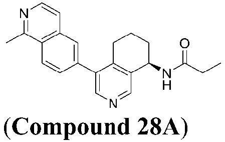 | (*R*)-*N*-(1'-methyl-5,6,7,8-tetrahydro-[4,6'-biisoquinolin]-8-yl)propionamide was obtained following the specific experimental procedures described in Example 1. [M+H]⁺ = 346.05. |
| **Example** 29 | **(*R*)-*N*-(3'-cyano-5,6,7,8-tetrahydro-[4,6'-biisoquinolin]-8-yl)propionamide** | 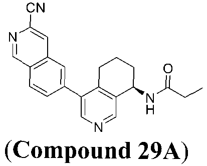 | (*R*)-*N*-(3'-cyano-5,6,7,8-tetrahydro-[4,6'-biisoquinolin]-8-yl)propionamide was obtained following the specific experimental procedures described in Example 1. [M+H]⁺ = 357.02. NMR data: ¹H NMR (400 MHz, DMSO-*d*₆) δ 9.55 (d, *J* = 1.0 Hz, 1H), 8.71 (d, *J* = 1.0 Hz, 1H), 8.45 (d, *J* = 0.7 Hz, 1H), 8.41 (d, *J* = 8.4 Hz, 1H), 8.37 (s, 1H), 8.33 (d, *J* = 8.4 Hz, 1H), 8.16 - 8.13 (m, 1H), 7.97 (dd, *J* = 8.4, 1.7 Hz, 1H), 5.15 (q, *J =* 6.6 Hz, 1H), 2.63 (t, *J=* 6.0 Hz, 3H), 2.19 (qd, *J=* 7.4, 5.1 Hz, 2H), 2.00 - 1.65 (m, 3H), 1.10 (d, *J* = 7.5 Hz, 3H). |
| **Example 30** | **(*R*)*-N*-(4-(2-cyclopropoxyquinolin-6-yl)-5,6,7,8-tetrahydroisoquinolin-8-yl)propionamide** | 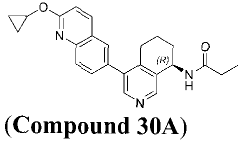 | (*R*)-*N*-(4-(2-cyclopropoxyquinolin-6-yl)-5,6,7,8-tetrahydroisoquinolin-8-yl)propionamide was obtained following the specific experimental procedures described in Example 1. [M+H]⁺ = 388.11. |
| **Example 31** | **(*R*)-*N*-(4-(2-(methylsulfonyl)quinolin-6-yl)-5,6,7,8-tetrahydroisoquinolin-8-yl)propionamide** | 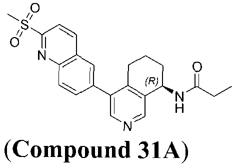 | (*R*)-*N*-(4-(2-(methylsulfonyl)quinolin-6-yl)-5,6,7,8-tetrahydroisoquinolin-8-yl)propionamide was obtained following the specific experimental procedures described in Example 27. [M+H]⁺ = 410.07. |
| **Example 32** | **(*R*)-*N*-(4-(2-hydroxyquinolin-6-yl)-5,6,7,8-tetrahydroisoquinolin-8-yl)propionamide** | 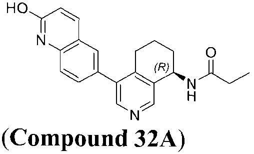 | (*R*)-*N*-(4-(2-hydroxyquinolin-6-yl)-5,6,7,8-tetrahydroisoquinolin-8-yl)propionamide was obtained following the specific experimental procedures described in Example 1. [M+H]⁺ =348.04. NMR data: ¹H NMR (400 MHz, DMSO-*d*₆) δ 11.86 (s, 1H), 8.34 (s, 1H), 8.28 (d, *J* = 8.4 Hz, 1H), 8.25 (s, 1H), 7.95 (d, *J =* 9.5 Hz, 1H), 7.67 (d, *J* = 1.9 Hz, 1H), 7.50 (dd, *J* = 8.4, 1.9 Hz, 1H), 7.39 (d, *J =* 8.4 Hz, 1H), 6.55 (d, *J* = 9.5 Hz, 1H), 5.10 (q, *J =* 5.7 Hz, 1H), 2.64-1.57 (m, 2H), 2.15 (qd, *J* = 7.4, 4.9 Hz, 2H), 1.94-1.83 (m, *J* = 8.8 Hz, 1H), 1.82-1.60 (m, 3H). 1.05 (t, *J* = 7.6 Hz, 3H). |
| **Example 33** | **(*R*)-*N*-(4-(3-methylquinolin-6-yl)-5,6,7,8-tetrahydroisoquinolin-8-yl)propionamide** | 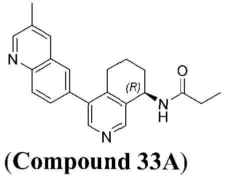 | (*R*)-*N*-(4-(3-methylquinolin-6-yl)-5,6,7,8-tetrahydroisoquinolin-8-yl)propionamide was obtained following the specific experimental procedures described in Example 1. [M+H]⁺ =346.02. NMR data: ¹H NMR (400 MHz, DMSO-*d*₆) δ 8.82 (d, *J* = 2.2 Hz, 1H), 8.41-8.37 (m, 1H), 8.3-8.27 (m, 2H), 8.18 (dd, *J =* 2.2, 1.1 Hz, 1H), 8.05 (d, *J =* 8.6 Hz, 1H), 7.87 (d, *J* = 2.1 Hz, 1H), 7.68 (dd, *J* = 8.6, 2.0 Hz, 1H), 5.13 (t, *J* = 6.9 Hz, 1H), 2.67-2.59 (m, 2H), 2.50(s, 4H), 2.26-2.09 (m, 2H), 1.90 (dd, *J* = 9.8, 4.8 Hz, 1H), 1.81-1.67 (m, 2H), 1.06 (t, *J* = 7.6 Hz, 3H). |

### Example 34

### Synthesis of (R)-N-(4-(3-fluoroquinolin-6-yl)-5,6,7,8-tetrahydroisoquinolin-8-yl)propionamide

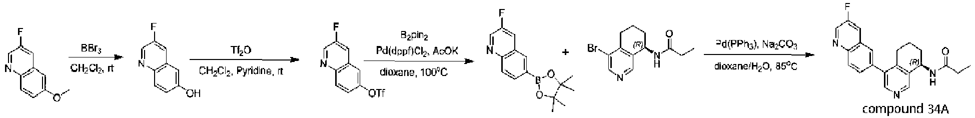

Specific synthetic scheme is as follows:

### Step A: Synthesis of 3-fluoroquinolin-6-ol

3-Fluoro-6-methoxyquinoline (400.0 mg, 2.26 mmol) was dissolved in dichloromethane (5 mL), to which was added a solution of boron tribromide in dichloromethane (23.0 mL, 1 mol/L) under nitrogen. The mixture was allowed to react at room temperature for 12 hours.

After the reaction was completed, methanol was added dropwise to the reaction mixture under ice-bath to quench the reaction. The mixture was concentrated under vacuum, and the resulting residue was purified by silica gel column chromatography (eluent: n-hexane/ethyl acetate = 0/1) to yield 230 mg of 3-fluoroquinolin-6-ol, as a white solid.

### Step B: Synthesis of 3-fluoroquinolin-6-yl trifluoromethanesulfonate

3-Fluoroquinolin-6-ol (180.0 mg, 1.13 mmol) and pyridine (450.0 mg, 5.65 mmol) were dissolved in dichloromethane (20 mL), to which was added trifluoromethanesulfonic anhydride (410.0 mg, 1.47 mmol) under ice-bath. The mixture was allowed to react at room temperature under a nitrogen atmosphere for 2 hours.

After the reaction was completed, water (10 mL) was added to the mixture, and the mixture was extracted with dichloromethane (10 mL×3). The organic phases were combined, washed with saturated brine (10 mL), dried over anhydrous sodium sulfate, and then concentrated under vacuum. The obtained residue was purified by silica gel column chromatography (eluent: n-hexane/ethyl acetate = 3/1) to obtain 124.0 mg of 3-fluoroquinolin-6-yl trifluoromethanesulfonate as a colorless liquid.

### Step C: Synthesis of 3-fluoro-6-(4,4,5,5-tetramethyl-1,3,2-dioxaborolan-2-yl)quinoline

3-fluoro-6-(4,4,5,5-tetramethyl-1,3,2-dioxaborolan-2-yl)quinoline was obtained following a synthesis method referred to Step C in Example 26. [M+H]⁺ = 274.08.

### Step D: Synthesis of (R)-N-(4-(3-fluoroquinolin-6-yl)-5,6,7,8-tetrahydroisoquinolin-8-yl)propionamide

Following a synthesis method similar to Step H in Example 1, (R)-4-bromo-5,6,7,8-tetrahydroisoquinolin-8-amine was coupled with 3-fluoro-6-(4,4,5,5-tetramethyl-1,3,2-dioxaborolan-2-yl)quinoline, to obtain (R)-N-(4-(3-fluoroquinolin-6-yl)-5,6,7,8-tetrahydroisoquinolin-8-yl)propionamide. [M+H]⁺ = 350.08.

### Example 35

### Synthesis of (R)-N-(4-(5-methylquinolin-6-yl)-5,6,7,8-tetrahydroisoquinolin-8-yl)propionamide

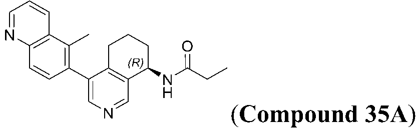

(*R*)-*N*-(4-(5-methylquinolin-6-yl)-5,6,7,8-tetrahydroisoquinolin-8-yl)propionamide was obtained following the specific experimental procedures described in Example 1. [M+H]⁺ =346.07. NMR data: ¹H NMR (400 MHz, DMSO-*d*₆) δ 8.96 (dd, *J =* 4.1, 1.6 Hz, 1H), 8.57 (d, *J =* 8.8 Hz, 1H), 8.40 (d, *J* = 2.5 Hz, 1H), 8.31 (dd, *J* = 8.4, 5.6 Hz, 1H), 8.19 (d, *J* = 5.0 Hz, 1H), 8.06-7.85 (m, 1H), 7.63 (dd, *J =* 8.6, 4.1 Hz, 1H), 7.47 (dd, *J =* 30.6, 8.6 Hz, 1H), 5.12 (t*, J =* 7.0 Hz, 1H), 2.38 (d, *J* = 10.0 Hz, 3H), 2.36-2.12 (m, 4H), 1.87 (s, 1H), 1.82-1.62 (m, 3H), 1.08 (td, *J =* 7.6, 2.4 Hz, 3H).

### Example 36

### Synthesis of (R)-N-(4-(5-fluoroquinolin-6-yl)-5,6,7,8-tetrahydroisoquinolin-8-yl)propionamide

Specific synthetic scheme is as follows:

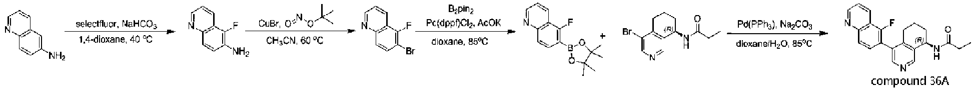

### Step A: Synthesis of 5-fluoroquinolin-6-amine

Quinolin-6-amine (721 mg, 5.0 mmol), sodium bicarbonate (1.26 g, 15.0 mmol), and Selectfluor (2.30 g, 6.5 mmol) were dissolved in 1,4-dioxane (25 mL), and the reaction mixture was stirred at 40°C for 10 hours.

After the reaction was completed, the mixture was filtered, and the filter cake was washed with acetonitrile (50 mL). The filtrate was collected, then dried over anhydrous sodium sulfate, and finally concentrated under reduced pressure. The obtained residue was purified by silica gel column chromatography (eluent: ethyl acetate/n-hexane = 1/5 to 1/3) to yield 330 mg of 5-fluoroquinolin-6-amine. [M+H]⁺ =163.19.

### Step B: Synthesis of 6-bromo-5-fluoroquinoline

5-Fluoroquinolin-6-amine (324 mg, 2.0 mmol) and cuprous bromide (344 mg, 2.4 mmol) were dissolved in acetonitrile (12 mL), stirred at 60°C for 0.5 h, then tert-butyl nitrite (268 mg, 2.6 mmol) was slowly added. The reaction mixture was stirred at 60°C for 8 hours, then cooled to room temperature and stirred for another 6 hours.

After the reaction was completed, to the reaction mixture was added 1N hydrochloric acid and stirred at room temperature for 1 hour, and then extracted with ethyl acetate (50 mL×3). The organic phases were combined, washed with saturated brine (25 mL), then dried over anhydrous sodium sulfate, and finally concentrated under reduced pressure. The obtained residue was purified by silica gel column chromatography (eluent: ethyl acetate/n-hexane = 1/4) to yield 50 mg of 6-bromo-5-fluoroquinoline. [M+H]⁺ =225.89.

### Step C: Synthesis of 5-fluoro-6-(4,4,5,5-tetramethyl-1,3,2-dioxaborolan-2-yl)quinoline

5-fluoro-6-(4,4,5,5-tetramethyl-1,3,2-dioxaborolan-2-yl)quinoline was obtained following the synthesis method referred to Step C in Example 26. [M+H]⁺ =274.24.

### Step D: Synthesis of (R)-N-(4-(5-fluoroquinolin-6-yl)-5,6,7,8-tetrahydroisoquinolin-8-yl)propionamide

Following a synthesis method similar to Step H in Example 1, (*R*)-4-bromo-5,6,7,8-tetrahydroisoquinolin-8-amine was coupled with 5-fluoro-6-(4,4,5,5-tetramethyl-1,3,2-dioxaborolan-2-yl)quinoline, to obtain (*R*)-*N*-(4-(5-fluoroquinolin-6-yl)-5,6,7,8-tetrahydroisoquinolin-8-yl)propionamide. [M+H]⁺ =350.22. NMR data: ¹H NMR (400 MHz, DMSO-*d*₆) δ 9.05 (dd, *J* = 4.2, 1.7 Hz, 1H), 8.55 (dt, *J* = 8.4, 1.3 Hz, 1H), 8.43 (s, 1H), 8.40-8.28 (m, 2H), 7.99 (d, *J* = 8.7 Hz, 1H), 7.72 (d, *J* = 4.2 Hz, 1H), 7.70 (d, *J* = 4.3 Hz, 1H), 5.14 (q, *J* = 6.6 Hz, 1H), 2.60-2.50 (m, 2H) 2.17 (qd, *J* = 7.4, 5.8 Hz, 2H), 2.00-1.60 (m, 4H), 1.07 (t, *J* = 7.6 Hz, 3H).

### Example 37

### Synthesis of (R)-N-(4-(5-cyanoquinolin-6-yl)-5,6,7,8-tetrahydroisoquinolin-8-yl)propionamide

Specific synthetic scheme is as follows:

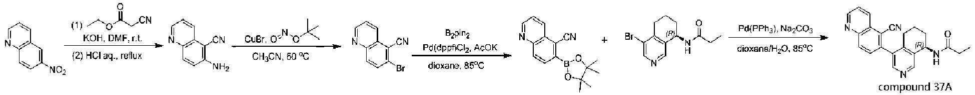

### Step A: Synthesis of 6-aminoquinolin-5-carbonitrile

6-Nitroquinoline (2.0 g, 11.48 mmol), ethyl 2-cyanoacetate (3.9 g, 26.52 mmol) and potassium hydroxide (1.9 g, 33.86 mmol) were added to N,N-dimethylformamide (30 mL), and stirred at room temperature overnight. The next day, the solvent was removed by evaporation under reduced pressure, and 10% aqueous hydrochloric acid solution (30 mL) was added. The mixture was refluxed for 3 hours.

After the reaction was completed, the reaction mixture was cooled and adjusted to pH 8 with saturated aqueous sodium hydroxide solution, and then extracted with ethyl acetate (30 mL×3). The organic phases were combined, washed with saturated brine (30 mL), then dried over anhydrous sodium sulfate, and finally concentrated under reduced pressure. The obtained residue was purified by silica gel column chromatography (eluent: dichloromethane/ethyl acetate = 1/3) to yield 1.9 g of 6-aminoquinoline-5-carbonitrile. [M+H]⁺ = 170.05.

### Step B: Synthesis of 6-bromoquinolin-5-carbonitrile

6-Aminoquinoline-5-carbonitrile (600 mg, 3.55 mmol) and cuprous bromide (609 mg, 4.26 mmol) were added to acetonitrile (12 mL). After 10 minutes, a solution of tert-butyl nitrite (474 mg, 4.61 mmol) in acetonitrile (1 mL) was added. Under nitrogen protection, the mixture was heated to 60°C and allowed to react for 8 hours, then allowed to stand at room temperature overnight. The next day, 1N aqueous hydrochloric acid solution (5 mL) was added and allowed to react for 3 hours.

After the reaction was completed, the mixture was extracted with ethyl acetate (20 mL×3). The organic phases were combined, washed with saturated brine (20 mL), then dried over anhydrous sodium sulfate, and finally concentrated under reduced pressure. The obtained residue was purified by silica gel column chromatography (eluent: n-hexane/ethyl acetate = 1/4) to yield 300 mg of 6-bromoquinoline-5-carbonitrile. [M+H]⁺ = 232.95.

### Step C: Synthesis of 6-(4,4,5,5-tetramethyl-1,3,2-dioxaborolan-2-yl)quinolin-5-carbonitrile

6-(4,4,5,5-tetramethyl-1,3,2-dioxaborolan-2-yl)quinolin-5-carbonitrile was obtained following the synthesis method referred to Step C in Example 26. [M+H]⁺ = 281.10.

### Step D: (R)-N-(4-(5-cyanoquinolin-6-yl)-5,6,7,8-tetrahydroisoquinolin-8-yl)propionamide

Following a synthesis method similar to Step H in Example 1, (*R*)-4-bromo-5,6,7,8-tetrahydroisoquinolin-8-amine was coupled with 6-(4,4,5,5-tetramethyl-1,3,2-dioxaborolan-2-yl)quinolin-5-carbonitrile, to obtian (*R*)-*N*-(4-(5-cyanoquinolin-6-yl)-5,6,7,8-tetrahydroisoquinolin-8-yl)propionamide, as a white solid. [M+H]⁺ = 357.04.

### Example 38

### Synthesis of (R)-N-(4-(8-cyanoquinolin-6-yl)-5,6,7,8-tetrahydroisoquinolin-8-yl)propionamide

Specific synthetic scheme is as follows:

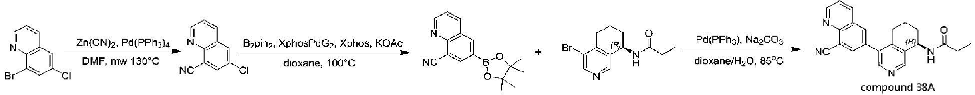

### Step A: Synthesis of 6-chloroquinolin-8-carbonitrile

8-Bromo-6-chloroquinoline (500 mg, 2.05 mmol), zinc cyanide (145 mg, 1.23 mmol) and tetrakis(triphenylphosphine)palladium (235 mg, 0.21 mmol) were added to N,N-dimethylformamide (5 mL). The mixture was reacted at 130°C for 45 minutes under microwave irradiation.

After the reaction was completed, the mixture was cooled and diluted with water, and then extracted with ethyl acetate (20 mL×3). The organic phases were combined, washed with saturated brine (30 mL), then dried over anhydrous sodium sulfate, and finally concentrated under reduced pressure. The obtained residue was purified by silica gel column chromatography (eluent: n-hexane/ethyl acetate = 3/1) to yield 380 mg of 6-chloroquinoline-8-carbonitrile. [M+H]⁺ = 189.14.

### Step B: Synthesis of 6-(4,4,5,5-tetramethyl-1,3,2-dioxaborolan-2-yl)quinolin-8-carbonitrile

Into dioxane (3 mL) were added 6-Bromoquinoline-5-carbonitrile (190 mg, 1.01 mmol), bis(pinacolato)diboron (381 mg, 1.50 mmol), chloro(2-dicyclohexylphosphino-2',4',6'-triisopropyl-1,1'-biphenyl)[2-(2'-amino-1,1'-biphenyl)]palladium(II) (79 mg, 0.10 mmol), 2-dicyclohexylphosphino-2',4',6'-triisopropylbiphenyl (48 mg, 0.10 mmol) and potassium acetate (294 mg, 3.00 mmol). The mixture was purged with nitrogen, heated to 100°C and allowed to react for 4 hours.

After the reaction was completed, the reaction mixture was diluted with water and then extracted with ethyl acetate (10 mL×3). The organic phases were combined, washed with saturated brine (10 mL), then dried over anhydrous sodium sulfate, and finally concentrated under reduced pressure. The obtained residue was purified by silica gel column chromatography (eluent: n-hexane/ethyl acetate = 1/2) to obtain 160 mg of 6-(4,4,5,5-tetramethyl-1,3,2-dioxaborolan-2-yl)quinoline-8-carbonitrile. [M+H]⁺ = 281.34.

### Step C: (R)-N-(4-(8-cyanoquinolin-6-yl)-5,6,7,8-tetrahydroisoquinolin-8-yl)propionamide

Following a synthesis method similar to Step H in Example 1, (*R*)-4-bromo-5,6,7,8-tetrahydroisoquinolin-8-amine was coupled with 6-(4,4,5,5-tetramethyl-1,3,2-dioxaborolan-2-yl)quinolin-8-carbonitrile, to obtain (R)-N-(4-(8-cyanoquinolin-6-yl)-5,6,7,8-tetrahydroisoquinolin-8-yl)propionamide, as a white solid. [M+H]⁺ = 357.03. NMR data: ¹H NMR (400 MHz, DMSO-*d₆*) *δ* 9.17 (dd, *J* = 4.2, 1.8 Hz, 1H), 8.63 (dd, *J* = 8.4, 1.8 Hz, 1H), 8.47 (d,*J =* 8.4 Hz, 2H), 8.41 (d, *J* = 4.2 Hz, 2H), 8.36 (d, *J =* 8.4 Hz, 1H), 7.82 (dd, *J* = 8.4, 4.2 Hz, 1H), 5.15 (d, *J* = 7.2 Hz, 1H), 2.69 (t, *J* = 6.0 Hz, 2H), 2.26-2.13 (m, 2H), 1.98-1.92 (m, 1H), 1.85-1.69 (m, 3H), 1.09 (t, *J =* 7.6 Hz, 3H).

### Example 39

### Synthesis of (R)-N-(4-(8-methoxyquinolin-6-yl)-5,6,7,8-tetrahydroisoquinolin-8-yl)propionamide

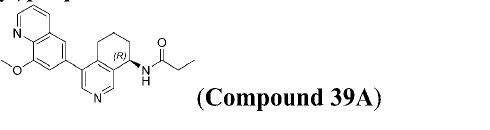

(*R*)-*N*-(4-(8-methoxyquinolin-6-yl)-5,6,7,8-tetrahydroisoquinolin-8-yl)propionamide was obtained following the specific experimental procedures described in Example 1. [M+H]⁺ = 362.06. NMR data: ¹H NMR (400 MHz, DMSO-*d*₆) *δ* 8.90 (dd, *J* = 4.2, 1.8 Hz, 1H), 8.41-8.31 (m, 3H), 8.32 (d, *J =* 8.4 Hz, 1H), 7.61 (dd, *J =* 8.4, 4.2 Hz, 1H), 7.49 (d, *J =* 1.8 Hz, 1H), 7.15 (d, *J =* 1.8 Hz, 1H), 5.15 (q, *J =* 6.6 Hz, 1H), 4.01 (s, 3H), 2.70 (t, *J =* 6.0 Hz, 2H), 2.25-2.12 (m, 2H), 1.96-1.87 (m, 1H), 1.86-1.68 (m, 3H), 1.09 (t, *J* = 7.6 Hz, 3H).

### Example 40

### Synthesis of (R)-N-(4-(quinolin-6-yl-2-d)-5,6,7,8-tetrahydroisoquinolin-8-yl)propionamide

Specific synthetic scheme is as follows:

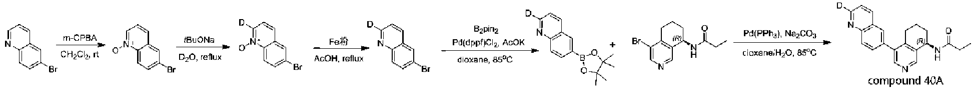

### Step A: Synthesis of 6-bromoquinolin-1-oxide

At room temperature, to a solution of 6-bromoquinoline (208 mg, 1.0 mmol) in dichloromethane (5 mL) was added m-chloroperoxybenzoic acid (304 mg, 1.5 mmol), and the mixture was stirred for 3 hours at that temperature.

After the reaction was completed, saturated sodium thiosulfate solution (20 mL) was added, and the mixture was stirred for 30 minutes. Then, 2N sodium hydroxide solution was added to adjust the pH to 9. The mixture were separated, and the organic phase was dried over anhydrous sodium sulfate, concentrated, and used directly in the next step. [M+H]⁺ = 223.96.

### Step B: Synthesis of 6-bromoquinolin-1-oxide-2d

At room temperature, to a solution of 6-bromoquinoline-1-oxide (224 mg, 1.0 mmol) in deuterium oxide (3 mL) was added sodium tert-butoxide (240 mg, 2.5 mmol). The mixture was stirred under reflux for 5 hours, and the reaction was monitored by LC-MS until completion.

After the reaction was completed, the mixture was diluted by adding water (20 mL), and then extracted with dichloromethane (20 mL×3). The combined organic phases were washed with saturated brine (20 mL×3), dried over anhydrous sodium sulfate, and concentrated directly for the next step. [M+H]⁺ = 224.92.

### Step C: Synthesis of 6-bromoquinolin-2-d

At room temperature, reduced iron powder (140 mg, 2.5 mmol) was added to a solution of 6-bromoquinoline-1-oxide-2-*d* (225 mg, 1.0 mmol) in acetic acid (3 mL). The mixture was stirred under reflux for 5 hours.

After the reaction was completed, the mixture was evaporated to remove the solvent, and then diluted by adding water (20 mL). Then 2N sodium hydroxide solution was added to adjust to pH 9. After phase separation, the organic layer was dried over anhydrous sodium sulfate, concentrated and filtered. The obtained solid was used directly in the next step. [M+H]⁺ = 209.02.

### Step D: Synthesis of 6-(4,4,5,5-tetramethyl-1,3,2-dioxaborolan-2-yl)quinolin-2-d

6-(4,4,5,5-tetramethyl-1,3,2-dioxaborolan-2-yl)quinolin-2-d was obtained following a synthesis method referred to Step C in Example 26. [M+H]⁺ = 257.10.

### Step E: Synthesis of (R)-N-(4-(quinolin-6-yl-2-d)-5,6,7,8-tetrahydroisoquinolin-8-yl)propionamide

Similar to a synthesis method similar to Step H in Example 1, (*R*)-4-bromo-5,6,7,8-tetrahydroisoquinolin-8-amine was coupled with 6-(4,4,5,5-tetramethyl-1,3,2-dioxaborolan-2-yl)quinolin-2-*d*, to obtain (*R*)-*N*-(4-(quinolin-6-yl-2-d)-5,6,7,8-tetrahydroisoquinolin-8-yl)propionamide. [M+H]⁺ = 333.03. NMR data: ¹H NMR (400 MHz, DMSO-*d*₆) δ 8.45 (d, *J* = 8.3 Hz, 1H), 8.42 (s, 1H), 8.36 (s, 1H), 8.33 (d, *J =* 8.4 Hz, 1H), 8.12 (d, *J =* 8.6 Hz, 1H), 8.01 (d, *J* = 1.9 Hz, 1H), 7.79 (dd, *J* = 8.6, 1.9 Hz, 1H), 7.62 (d, *J* = 8.3 Hz, 1H), 5.15 (q, *J =* 6.6 Hz, 1H), 2.69-2.63 (m, 2H), 2.30-2.12 (m, 2H), 1.97-1.65 (m, 4H), 1.09 (t, *J =* 7.6 Hz, 3H).

### Example 41-43

| | Chemical Name | Structure | Preparation and characterization |
|---|---|---|---|
| **Example 41** | **(*R*)-*N*-(4-(imidazo[1,5-a]pyridin-7-yl)-5,6,7,8-tetrahydroisoquinolin-8-yl)propionamide** | 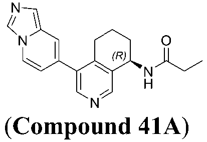 | (*R*)-*N*-(4-(imidazo[1,5-a]pyridin-7-yl)-5,6,7,8-tetrahydroisoquinolin-8-yl)propionamide was obtained following the specific experimental procedures described in Example 1. [M+H]⁺ = 321.11. NMR data: ¹H NMR (400 MHz, DMSO-*d*₆) δ 8.45 (s, 1H), 8.44-8.40 (m, 1H), 8.37 (s, 1H), 8.30 (d, *J* = 9.8 Hz, 2H), 7.55 (s, 1H), 7.43 (s, 1H), 6.71 (dd, *J* = 7.3, 1.7 Hz, 1H), 5.16-5.10 (m, 1H), 2.67 (s, 2H), 2.23-2.12 (m, 2H), 1.95-1.79 (m, 2H), 1.79-1.69 (m, 2H), 1.08 (t, *J* = 7.6 Hz, 3H). |
| **Example 42** | **(*R*)-*N*-(4-(imidazo[1,2-a]pyridin-7-yl)-5,6,7,8-tetrahydroisoquinolin-8-yl)propionamide** | 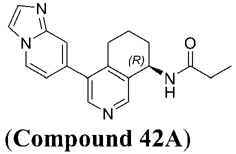 | (*R*)-*N*-(4-(imidazo[1,2-a]pyridin-7-yl)-5,6,7,8-tetrahydroisoquinolin-8-yl)propionamide was obtained following the specific experimental procedures described in Example 1. [M+H]⁺ =321.02. |
| **Example 43** | **(*R*)*-N*-(4-(6-cyanonaphthalen-2-yl)-5,6,7,8-tetrahydroisoquinolin-8-yl)propionamide** | 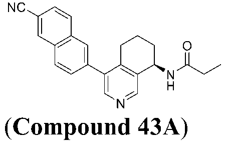 | (*R*)-*N*-(4-(6-cyanonaphthalen-2-yl)-5,6,7,8-tetrahydroisoquinolin-8-yl)propionamide was obtained following the specific experimental procedures described in Example 1. [M+H]⁺ =356.06. |

### Example 44-46

Compounds 44A-46A were prepared following the synthesis method described for the preparation of the aforementioned compounds.

| 44A | 45A | 46A |
|---|---|---|
| 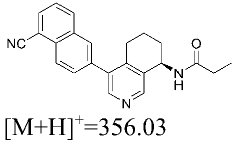 | 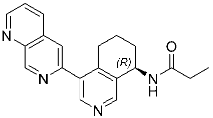 | 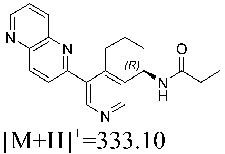 |

### Example 47

### Synthesis of 4-(quinolin-6-yl)-5,6,7,8-tetrahydroisoquinolin-8-ol

Specific synthetic scheme is as follows:

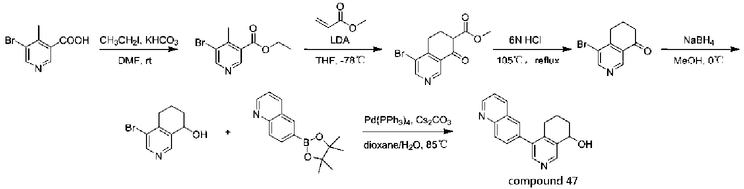

### Step A: Synthesis of 4-bromo-6,7-dihydroisoquinolin-8(5H)-one

4-Bromo-6,7-dihydroisoquinolin-8(5H)-one was prepared according to the procedures described in Example 1. [M+H]⁺ = 226.05.

### Step B: Synthesis of 4-bromo-5,6,7,8-tetrahydroisoquinolin-8-ol

At 0°C, sodium borohydride (0.39 g, 10.4 mmol) was added in portions to a solution of 4-bromo-6,7-dihydroisoquinolin-8(5H)-one (1.96 g, 8.67 mmol) in methanol (20 mL). The mixture was stirred at 0°C for another 20 minutes.

After the reaction was completed, the reaction was quenched by adding 10 mL of water, and the solvent was removed by rotary evaporation. Water (30 mL) was added to the residue, and the mixture was extracted with ethyl acetate (30 mL × 3). The organic phases were combined, washed with saturated brine (30 mL), dried over anhydrous sodium sulfate, and finally concentrated under reduced pressure. The obtained residue was purified by silica gel column chromatography (eluent: methanol/dichloromethane = 1/10) to obtain 1.94 g of 4-bromo-5,6,7,8-tetrahydroisoquinolin-8-ol. [M+H]⁺ = 228.02.

### Step C: Synthesis of 4-(quinolin-6-yl)-5,6,7,8-tetrahydroisoquinolin-8-ol

4-Bromo-5,6,7,8-tetrahydroisoquinolin-8-ol (100 mg, 0.44 mmol) and 1-methyl-6-(4,4,5,5-tetramethyl-1,3,2-dioxaborolan-2-yl)-3,4-dihydroquinolin-2(1H)-one (134 mg, 0.53 mmol) were dissolved in a mixture of dioxane (100 mL) and water (20 mL), to which were added cesium carbonate (285 mg, 0.88 mmol) and tetrakis(triphenylphosphine)palladium (25 mg, 0.022 mmol), and the mixture was allowed to react at 85°C for 6 hours under a nitrogen atmosphere.

After the reaction was completed, the obtained suspension was filtered. The filter cake was washed with dichloromethane, and the filtrate was concentrated under reduced pressure. The obtained residue was purified by silica gel column chromatography (eluent: methanol/dichloromethane = 1/30) to obtain 149 mg of 4-(quinolin-6-yl)-5,6,7,8-tetrahydroisoquinolin-8-ol. [M+H]⁺ = 277.11. ¹H NMR (400 MHz, DMSO-d₆) δ 8.97 (dd, J = 4.2, 1.7 Hz, 1H), 8.64 (s, 1H), 8.43 (d, J = 7.9 Hz, 1H), 8.34 (s, 1H), 8.11 (d, J = 8.6 Hz, 1H), 8.00 (d, J = 2.0 Hz, 1H), 7.79 (dd, J = 8.6, 2.0 Hz, 1H), 7.60 (dd, J = 8.3, 4.2 Hz, 1H), 5.44 (d, J = 5.5 Hz, 1H), 4.78 (q, J = 5.5 Hz, 1H), 2.70-2.54 (m, 2H), 1.98-1.75 (m, 3H), 1.69-1.59 (m, 1H).

### Examples 48 and 49

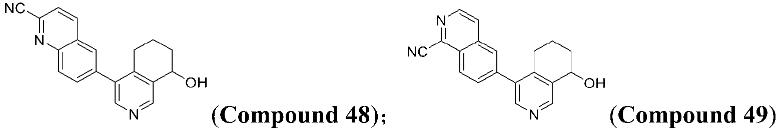

Compounds 48 and 49 were synthesized following the procedures described in Example 47. Specific structure and characterization of the compounds is as follows:
**Compound 48:** [M+H]⁺ = 302.17; ¹H NMR (400 MHz, DMSO-d6) δ 8.72 (d, J = 8.4 Hz, 1H), 8.66 (s, 1H), 8.36 (s, 1H), 8.23 (d, J = 8.8 Hz, 1H), 8.18 (d, J = 1.9 Hz, 1H), 8.12 (d, J = 8.4 Hz, 1H), 7.99 (dd, J = 8.7, 2.0 Hz, 1H), 5.46 (d, J = 5.5 Hz, 1H), 4.79 (q, J = 5.5 Hz, 1H), 2.71-2.55 (m, 2H), 1.99-1.75 (m, 3H), 1.69-1.60 (m, 1H).
**Compound 49:** [M+H]⁺ = 302.12; ¹H NMR (400 MHz, DMSO-d6) δ 8.79 (d, J = 5.6 Hz, 1H), 8.68 (s, 1H), 8.38-8.30 (m, 3H), 8.25 (d, J = 1.6 Hz, 1H), 8.00 (dd, J = 8.5, 1.7 Hz, 1H), 5.47 (d, J = 5.5 Hz, 1H), 4.79 (q, J = 5.5 Hz, 1H), 2.70-2.56 (m, 2H), 2.00-1.75 (m, 3H), 1.70-1.60 (m, 1H).

### Example 50: Biological Activity Evaluation

### Detection Method

The H295R steroidogenesis assay system was used in the present invention for testing the enzyme activities of human CYP11B1, human CYP11B2, etc. The in vitro H295R steroidogenesis assay system utilizes a human adrenocortical carcinoma cell line (NCI-H295R cells) as a Tier 2 "in vitro assay that provides mechanistic data" for screening and prioritization purposes. The development and standardization of this method were performed in a multi-step process for screening chemical effects on steroidogenesis, and the H295R assay method has been optimized and validated according to the OECD Test Guideline (Test Guideline No. 456 H295R Steroidogenesis Assay).

### Inhibition of Aldosterone Synthase

NCI-H295R cells were purchased from ATCC. After initiating culture from the original ATCC batch, NCI-H295R cells were cultured for five passages (i.e., undergo four rounds of cell divisions), and then the cells at passage five were cryopreserved and stored in liquid nitrogen.

H295R cells were cultured in a cell culture incubator at 37°C and 5% CO₂, and the medium was replaced 2-3 times per week. Cells were passaged when they reached approximately 85-90% confluency. The medium was aspirated, and the cells were washed three times with DPBS (Ca²⁺ and Mg²⁺ -free), and then trypsin was added to digest for 1-3 min. The digestion was terminated by adding 3 mL of culture medium. The cells were then dislodged by pipetting, and any remaining cells were rinsed off with 1 mL of medium. The cell suspension was pooled and transferred to a 15 mL centrifuge tube. After centrifuging at room temperature and 800 rpm for 5 min, the supernatant was discarded, the pellet was resuspended in 3 mL of medium, and the cell suspension was counted. The edge wells of a 96-well plate are left empty, and the remaining wells are seeded with 50,000 cells per well in 100 µL of 10% FBS DMEM/F12 (1:1) basal medium..After overnight recovery, the medium was replaced with basal medium containing 10 µM Forskolin (150 µL per well), and the cells were incubated for 48 h. After 48 h, the medium was replaced with basal medium containing 10 µM deoxycorticosterone. The compound was dissolved in DMSO to prepare a 100 mM stock solution. Starting from 100 mM, the compounds are subjected to 3-fold serial dilutions in DMSO, resulting in 10 concentration points. These 10 concentration points was then diluted 10-fold with blank DMEM:F12 (1:1) medium, resulting in a starting concentration of 10 mM. An aliquot of 1.5 µL of each compound concentration was added to the cells, achieving a final DMSO concentration of 0.1% and a starting compound concentration of 100 µM. After incubation for 48 h, 40 µL of cell supernatant was collected and analyzed for the levels of aldosterone and cortisol by LC-MS.

### Cell Viability Assay

After collecting the supernatant, 100 µL of 10% CCK8 detection reagent was added to each well, and the plate was incubated at 37°C for 10 min. The plate was gently tapped to mix, and the OD value was measured at 405 nm using a microplate reader. The 70% methanol group was used as the negative control, and the DMSO solvent control served as the positive control. Cell viability was calculated using the following formula: 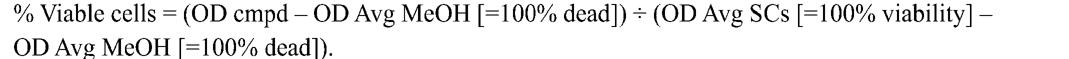 % Viable cells = (OD cmpd - OD Avg MeOH [=100% dead]) ÷ (OD Avg SCs [=100% viability] - OD Avg MeOH [=100% dead]).

Wells with cell viability below 80% should be excluded from the final data analysis. When cytotoxicity of approximately 20% is present, the inhibition of steroidogenesis should be carefully evaluated to ensure that cytotoxicity is not the cause of the observed inhibition. Additionally, if cell viability exceeds 120%, the data should be marked to identify potential false positives.

### Result Analysis

The inhibition rate was calculated using the following formula: 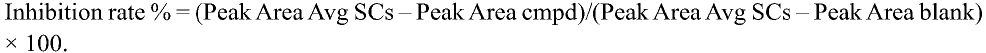 Inhibition rate % = (Peak Area Avg SCs - Peak Area cmpd)/(Peak Area Avg SCs - Peak Area blank) × 100.

Using the logarithm of the compound concentration as the x-axis and the inhibition rate as the y-axis. Nonlinear regression curve fitting was performed using Graphpad 9.0 to calculate the IC₅₀ value (Y=Bottom+(Top - Bottom)/(1+ 10^((LogIC₅₀ - X)×HillSlope))), Ki = IC₅₀/(1 + [S]/Km), and the test results are shown in Table 1. Unless otherwise specified, competitive inhibition is assumed for all proteases. Selectivity = CYP11B1 Ki (nM)/CYP11B2 Ki (nM); wherein, A represents a selectivity value between 0 and 50, B represents a selectivity value between 51 and 100, C represents a selectivity value between 101 and 150, and D represents a selectivity value above 151.

**Table 1: Inhibitory effect of compounds on CYP11B2**

| Example compound No. | CYP11B2 Ki (nM) | Selectivity |
|---|---|---|
| **1A** | <50 | B |
| **2A** | <50 | B |
| **7A** | <50 | A |
| **8A** | <50 | A |
| **14A** | <50 | A |
| **19A** | <50 | A |
| **26A** | <50 | B |
| **34A** | <50 | A |
| **36A** | <50 | A |
| **40A** | <50 | A |
| **43A** | <50 | A |
| **47** | <50 | A |
| **48** | <50 | A |

Based on the experimental results in Table 1, the compounds of the present invention exhibit favorable inhibitory activity against CYP11B2, with superior efficacy compared to the control compound Baxdrostat. Furthermore, the compounds of the present invention exhibit excellent selectivity for CYP11B2, effectively inhibiting CYP11B2 while showing a relatively weaker inhibition of CYP11B1.

### Example 51: Rat Pharmacokinetic Study

### Experimental Materials

SD rats: male, 180-250g, purchased from Beijing Vital River Laboratory Animal Technology Co., Ltd.
Reagents: DMSO (dimethyl sulfoxide), PEG-400 (polyethylene glycol 400), saline, heparin, acetonitrile, formic acid, and propranolol (internal standard) are all commercially available. Instrument: AB SCIEX QTRAP 5500+.

### Experimental Method

The compounds of Examples 1-49 of the present invention were weighed and dissolved in a DMSO-PEG-400-saline (5:60:35, v/v/v) system. Following intravenous or intragastric administration to rats, venous blood samples (200 µL) were collected into EDTA-K2 anticoagulant tubes at the following time points: 15 min, 30 min, 1 h, 2 h, 5 h, 7h, and 24 h (with an additional 5-min time point for the i.v. group). The samples were centrifuged at 12,000 rpm for 2 minutes, and the obtained plasma was stored at -80°C until analysis. A certain amount of the test sample was accurately weighed and dissolved in DMSO to 2 mg/mL as a stock solution. An appropriate amount of the stock solution of each test compound was accurately pipetted and diluted with acetonitrile to prepare a series of standard solutions. 10 µL of each of the above series of standard solutions was accurately pipetted, to which was added 90 µL of blank plasma, and vortexed to prepare plasma samples with plasma concentrations equivalent to 1, 3, 5, 10, 30, 100, 300, 1000, 3000 ng/mL. Duplicate samples were analyzed for each concentration to establish a standard curve. 30 µL aliquot of each plasma sample was used (samples from the 5, 15, and 30 min i.v. time points were diluted 5-fold prior to analysis). Then, 150 µL of acetonitrile containing the internal standard propranolol (50 ng/mL) was added. After vortexing, 100 µL of purified water was added, and the mixture was vortexed again, followed by centrifugation at 4000 rpm for 5 minutes. The supernatant was collected and analyzed by LC-MS.

The LC-MS analysis was performed under the following conditions:
Chromatographic column: YMC Triart C18, 50×3.0mm, 2.1µm.
Mobile phase: gradient elution with a mixture of water (containing 0.1% formic acid) and acetonitrile as described in the table below:

| Time (min) | water (containing 0.1% formic acid) | acetonitrile |
|---|---|---|
| 0 | 80% | 20% |
| 0.6 | 80% | 20% |
| 1.2 | 15% | 85% |
| 2.6 | 15% | 85% |
| 2.61 | 80% | 20% |
| 3.2 | 80% | 20% |

### Data Processing

After the plasma concentration was determined by LC-MS, the pharmacokinetic parameters were calculated using WinNonlin 6.1 software with a non-compartmental model. The results are shown in Table 2.

**Table 2 Pharmacokinetic results of the compounds of the present invention in rats**

| | Dose (mg/kg) | Cₘₐₓ | AUCₗₐₛₜ | T_{1/2} |
|---|---|---|---|---|
| Compound ID | Route of administration | (ng/ml) | (h*ng/ml) | (h) |
| **Baxdrostat** | 1, i.v | 1270 | 5050 | 3.92 |
| | 5, p.o | 2980 | 28400 | 4.37 |
| **8A** | 1, i.v | 2850 | 7910 | 2.05 |
| | 5, p.o | 6280 | 53800 | 2.50 |
| **26A** | 1, i.v | 3360 | 9650 | 3.06 |
| | 5, p.o | 4620 | 42600 | 2.57 |

Based on the experimental results in Table 2, it can be observed that the compounds of the present invention exhibit favorable pharmacokinetic profiles in SD rats. Both Cmax and AUClast following intravenous and intragastric administration are superior to those of the positive control, indicating good absorption and high absolute bioavailability. The half-life is comparable to or better than that of the control compound.

### Example 52: In Vitro Hepatic Microsome Stability Study in Different Animal Species

### Preparation of Stock Solutions and Working Solutions

The compounds of Examples 1-49 (test substances) and the positive control drug were dissolved in DMSO to prepare stock solutions (10 mM). The stock solutions were diluted with acetonitrilewater (1:1, v/v) to obtain 100 µM solutions, which was further diluted with 0.1 M potassium phosphate buffer to obtain working solutions (30 µM).

NADPH powder was weighed and dissolved in 0.1 M potassium phosphate buffer to obtain a 5 mg/mL solution.

Hepatic microsomes from each species (20 mg/mL) were diluted with 0.1 M potassium phosphate buffer to parepare 0.8 mg/mL hepatic microsome working solutions.

### Hepatic Microsome Stability Determination

25 µL of the test substance or positive control drug working solution was added to 475 µL of the hepatic microsome working solution and mixed thoroughly. The mixture was aliquoted into a 96-well plate at 30 µL/well (n=2). For the 0 min samples, 150 µL of internal standard acetonitrile solution was added to precipitate proteins, followed by the addition of 15 µL of NADPH solution, and these samples were then stored in a refrigerator at 4°C. Other samples were pre-incubated at 37°C for 10 min. For the 20 min and 60 min samples, 15 µL of NADPH solution was added to initiate the reaction. For samples without NADPH, 15 µL of potassium phosphate buffer was added instead. All samples were then incubated at 37°C. Upon reaching the designated reaction time, 150 µL of internal standard acetonitrile solution was added to precipitate proteins.

The precipitated samples were vortexed and then centrifuged at 4000 rpm for 5 min. 100 µL of purified water was added to the supernatant , followed by LC-MS analysis.

### Data Analysis

The peak area ratio of the analyte to the internal standard was used to calculate the relative percentage of the compound remaining after incubation (remaining rate %) and for exponential function fitting. The calculation formula was as follows: 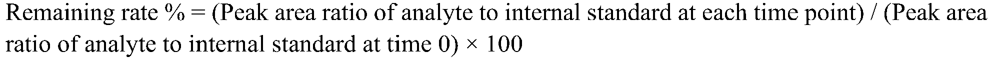 Remaining rate % = (Peak area ratio of analyte to internal standard at each time point) / (Peak area ratio of analyte to internal standard at time 0) × 100 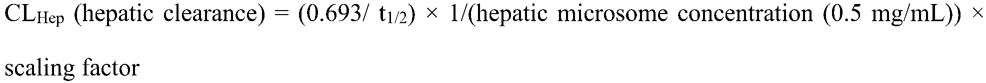 CLHep (hepatic clearance) = (0.693/ t1/2) × 1/(hepatic microsome concentration (0.5 mg/mL)) × scaling factor 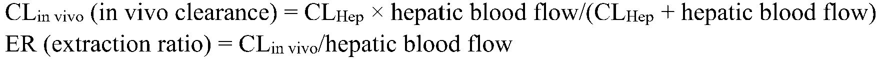 CLin vivo (in vivo clearance) = CLHep × hepatic blood flow/(CLHep + hepatic blood flow) ER (extraction ratio) = CLin vivo/hepatic blood flow

### Physiological parameters

| Species | Microsomal protein (mg/g Liver weight) | Liver weight (g/kg, body weight) | Scaling factor | Hepatic blood flow (ml/min/kg) |
|---|---|---|---|---|
| Human | 48.8 | 25.7 | 1254.2 | 20.7 |

| | | | | |
|---|---|---|---|---|
| Classification criteria: slow metabolism (ER<0.3), moderate metabolism (0.3<ER<0.7), fast metabolism (ER>0.7). | | | | |

The test results are shown in Table 3.

**Table 3 Stability of the compounds in human hepatic microsomes**

| **Compound ID** | **Human** | |
|---|---|---|
| | **T_{1/2}(min)** | **ER** |
| **Baxdrostat** | 690 | 0.11 |
| **1A** | >2000 | <0.04 |
| **20A** | 2097 | 0.04 |
| 26A | 1908 | 0.04 |

The experimental results in Table 3 demonstrated that the compounds of the present invention exhibit better stability in human hepatic microsomes than the positive control drug Baxdrostat.

### Example 53: Pharmacokinetic Study of Compounds in Cynomolgus Monkeys

### Experimental Materials

Cynomolgus monkeys: male, 180-250g, purchased from Guangxi Grandforest Scientific Primate Company, Ltd.
Reagents: DMSO (dimethyl sulfoxide), PEG400, saline, heparin, acetonitrile, formic acid, propranolol (internal standard) are all commercially available.
Instrument: AB SCIEX 7500.

### Experimental Method

The compound was weighed and dissolved in a DMSO-PEG-400-saline (5:60:35, v/v/v) system. Following intragastric administration to cynomolgus monkeys, 200 µL of venous blood was collected into EDTA-K2 anticoagulant tubes at 30 min, 60 min, 90 min, 2 h, 3 h, 5 h, 8 h, and 24 h. The blood samples were centrifuged at 12,000 rpm for 2 min, and the plasma was collected and stored at -80°C for subsequent analysis. An appropriate amount of the test compound was accurately weighed and dissolved in DMSO to a concentration of 2 mg/mL as a stock solution. Appropriate volumes of the compound stock solution were accurately pipetted and diluted with acetonitrile to prepare a series of standard solutions. A series of standard solutions were prepared at concentrations equivalent to plasma concentrations of 0.3, 1, 3, 10, 30, 100, 300, 1000, and 3000 ng/mL by accurately pipetting 10 µL of each standard solution and adding it to 90 µL of blank plasma, followed by vortexing to mix. Quality control samples were prepared at plasma concentrations of 2.4, 120, and 2400 ng/mL. Duplicate samples were analyzed for each concentration , and the standard curve was established. Subsequently, 30 µL aliquot of plasma was taken, and 150 µL of acetonitrile solution containing the internal standard propranolol (50 ng/mL) was added. After vortexing to mix, 100 µL of purified water was added, and the mixture was vortexed again to mix. It was then centrifuged at 4000 rpm for 5 minutes, and the supernatant was collected for LC-MS analysis. The LC-MS detection conditions were as follows:
Chromatographic column: YMC Triart C18, 50×3.0mm, 2.1µm.
Mobile phase: gradient elution with a mixture of water (containing 0.1% formic acid) and acetonitrile as described in the table below:

| Time (min) | water (containing 0.1% formic acid) | acetonitrile |
|---|---|---|
| 0 | 80% | 20% |
| 0.6 | 80% | 20% |
| 1.2 | 15% | 85% |
| 2.6 | 15% | 85% |
| 2.61 | 80% | 20% |
| 3.2 | 80% | 20% |

### Data Processing

After the plasma concentration was determined by LC-MS, the pharmacokinetic parameters were calculated using WinNonlin 6.1 software with a non-compartmental model, and the results are shown in Table 4.

**Table 4 Pharmacokinetic results of the compounds of the present invention in Cynomolgus monkey**

| Compound ID | Dose (mg/kg); Route of administration | Cₘₐₓ (ng/ml) | AUCₗₐₛₜ (h*ng/ml) | T_{1/2} (h) |
|---|---|---|---|---|
| **Baxdrostat** | 0.05, ig | 18.9 | 121 | 5.52 |
| | 20, ig | 7157 | 46238 | 4.08 |
| **8A** | 0.05, ig | 14.3 | 122 | 8.55 |
| | 20, ig | 7800 | 68600 | 7.06 |
| **26A** | 0.05, ig | 33.8 | 292 | 6.39 |
| | 20, ig | 15000 | 128500 | 5.63 |

Based on the experimental results in Table 4, it can be observed that the series of compounds of the present invention exhibited favorable pharmacokinetic profiles in cynomolgus monkeys. Following intragastric administration, the exposure levels after absorption were higher than or comparable to those of the positive control Baxdrostat, with both Cmax and AUClast being superior to the positive control. The half-life also demonstrated an advantage, indicating good absorption and high absolute bioavailability.

It should be understood that the above examples are preferable embodiments of the present invention, but the embodiments of the present invention are not limited by the above examples. For those of ordinary skill in the art, modifications or variations can be made based on the above description, and all such modifications and variations shall fall within the protection scope of the appended claims of the present invention.

## Claims

1. A compound, an isomer thereof, a racemate thereof, or a pharmaceutically acceptable salt thereof, **characterized in that** the structure of the compound is as shown in Formula I or Formula IA: 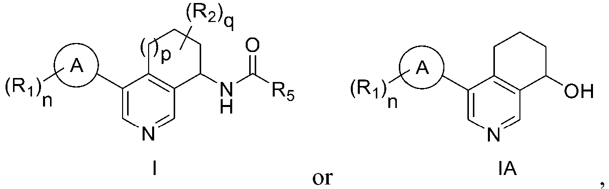 wherein,
R₁ and R₂ are each independently selected from H, halogen, hydroxyl, cyano, substituted or unsubstituted C₁-C₈ alkyl, substituted or unsubstituted C₁-C₈ alkoxy, substituted or unsubstituted C₃-C₈ cycloalkyl, substituted or unsubstituted 3-8 membered heterocycloalkyl, C₁-C₆ alkylsulfonyl, substituted or unsubstituted C₆-C₁₂ aryl or substituted or unsubstituted 5-12 membered heteroaryl;
R₅ is independently selected from substituted or unsubstituted C₁-C₈ alkyl, substituted or unsubstituted C₁-C₈ alkoxy, substituted or unsubstituted C₃-C₈ cycloalkyl, substituted or unsubstituted 3-8 membered heterocycloalkyl, substituted or unsubstituted C₆-C₁₂ aryl or substituted or unsubstituted 5-12 membered heteroaryl;
ring A is independently selected from 5-12 membered fused heteroaryl or C₁₀-C₁₂ aryl;
the substituents in the above "substituted" are each independently selected from one or more of C₁-C₈ alkyl, halo C₁-C₈ alkyl, C₁-C₈ alkoxy, halo C₁-C₈ alkoxy, -NR₃R₄, hydroxyl, oxo, carboxyl, cyano, halogen, C₁-C₆ alkylsulfonyl, C₁-C₆ alkylacyl, C₁-C₆ alkylamido, C₃-C₈ cycloalkyl, C₃-C₈ cycloalkyloxy, 3-8 membered heterocycloalkyl, 3-8 membered heterocycloalkyloxy, C₃-C₈ cycloalkyl-substituted C₁-C₈ alkoxy, C₆-C₁₂ aryl, C₆-C₁₂ aryloxy, C₁-C₈ alkyl-substituted C₆-C₁₂ aryloxy, C₁-C₈ alkoxy-substituted C₆-C₁₂ aryloxy, halo C₁-C₈ alkyl-substituted C₆-C₁₂ aryloxy, 5-12 membered heteroaryl, 5-12 membered heteroaryloxy, C₁-C₈ alkyl-substituted 5-12 membered heteroaryloxy, C₁-C₈ alkoxy-substituted 5-12 membered heteroaryloxy or halo C₁-C₈ alkyl-substituted 5-12 membered heteroaryloxy;
R₃ and R₄ are each independently selected from H or C₁-C₈ alkyl;
n, p or q are each independently selected from an integer of 0, 1, 2 or 3.

2. The compound, an isomer thereof, a racemate thereof, or a pharmaceutically acceptable salt thereof according to claim 1, **characterized in that** the structure of the compound is as shown in Formula IIIA, Formula IIIB, Formula IIIC or Formula IIID: 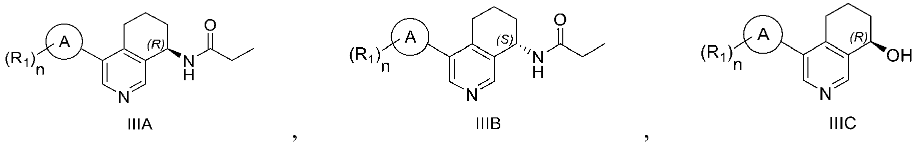 or 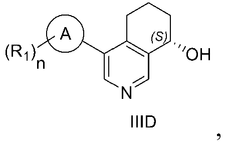 wherein, the definitions of R₁, ring A and n are the same as in claim 1.

3. The compound, an isomer thereof, a racemate thereof, or a pharmaceutically acceptable salt thereof according to any one of claims 1-2, **characterized in that** ring A is selected from 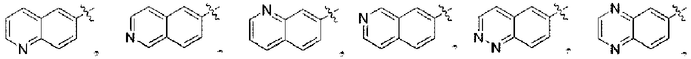 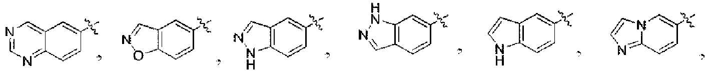 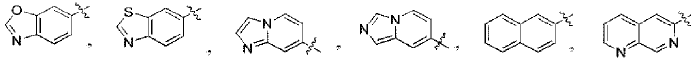 or

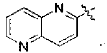

4. The compound, an isomer thereof, a racemate thereof, or a pharmaceutically acceptable salt thereof according to any one of claims 1-3, **characterized in that** R₁ and R₂ are each independently selected from H, hydroxyl, halogen, cyano, C₁-C₆ alkyl, halo C₁-C₆ alkyl, C₁-C₆ alkoxy, halo C₁-C₆ alkoxy, C₃-C₈ cycloalkyl, C₃-C₈ cycloalkyloxy, C₁-C₆ alkylsulfonyl, C₁-C₆ alkylacyl, 3-8 membered heterocycloalkyl, C₆-C₁₀ aryl or 5-10 membered heteroaryl.

5. The compound, an isomer thereof, a racemate thereof, or a pharmaceutically acceptable salt thereof according to any one of claims 1-4, **characterized in that** R₁ and R₂ are each independently selected from H, hydroxyl, F, Cl, cyano, methyl, methoxy, trifluoromethyl, methylsulfonyl, or cyclopropyloxy.

6. The compound, an isomer thereof, a racemate thereof, or a pharmaceutically acceptable salt thereof according to any one of claims 1-5, **characterized in that** the compound is selected from the following compounds:
| No. | Structure | No. | Structure | No. | Structure |
|---|---|---|---|---|---|
| 1 | 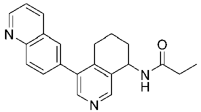 | 1A | 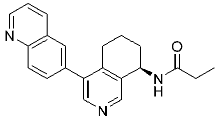 | 1B | 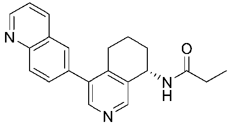 |
| 2 | 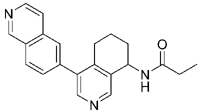 | 2A | 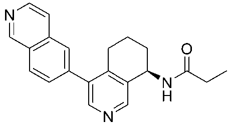 | 2B | 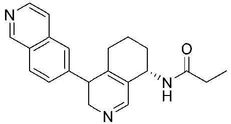 |
| 3 | 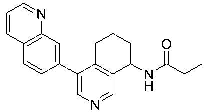 | 3A | 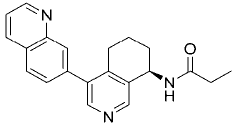 | 3B | 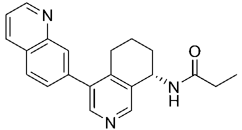 |
| 4 | 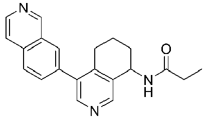 | 4A | 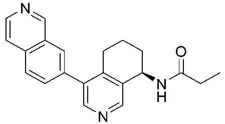 | 4B | 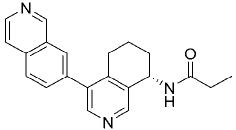 |
| 5 | 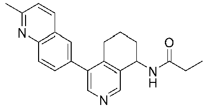 | 5A | 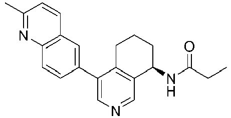 | 5B | 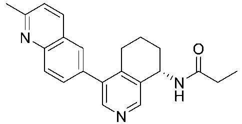 |
| 6 | 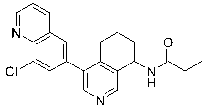 | 6A | 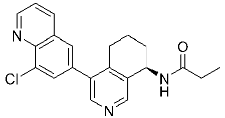 | 6B | 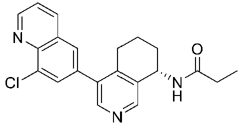 |
| 7 | 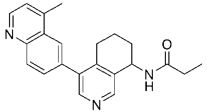 | 7A | 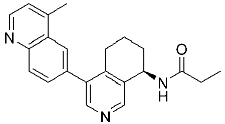 | 7B | 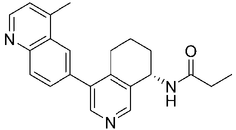 |
| 8 | 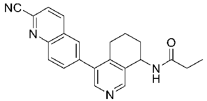 | 8A | 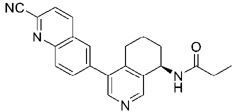 | 8B | 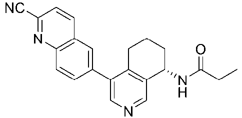 |
| 9 | 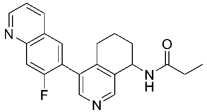 | 9A | 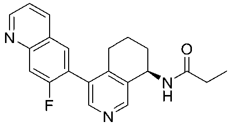 | 9B | 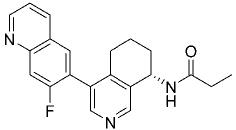 |
| 11 | 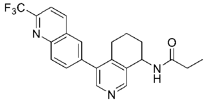 | 11A | 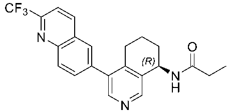 | 11B | 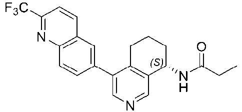 |
| 12 | 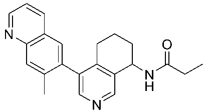 | 12A | 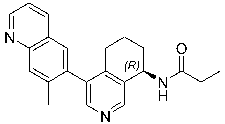 | 12B | 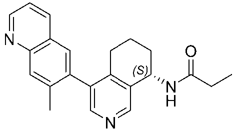 |
| 13 | 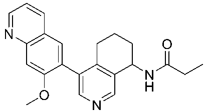 | 13A | 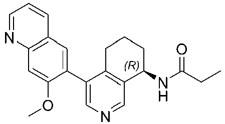 | 13B | 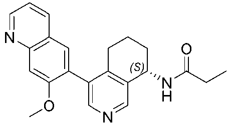 |
| 14 | 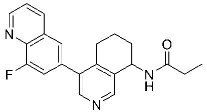 | 14A | 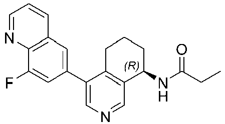 | 14B | 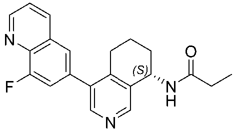 |
| 15 | 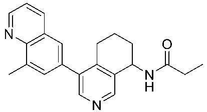 | 15A | 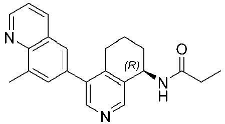 | 15B | 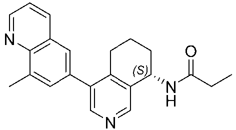 |
| 16 | 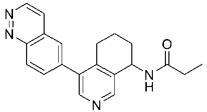 | 16A | 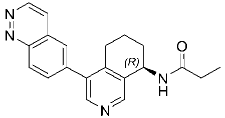 | 16B | 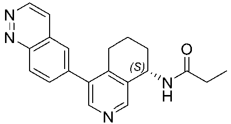 |
| 17 | 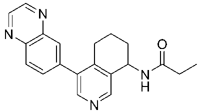 | 17A | 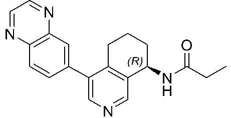 | 17B | 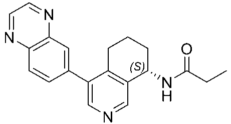 |
| 18 | 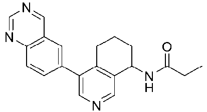 | 18A | 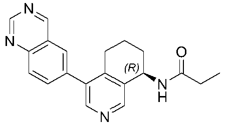 | 18B | 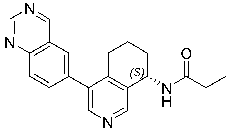 |
| 19 | 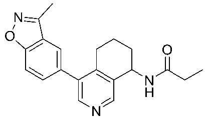 | 19A | 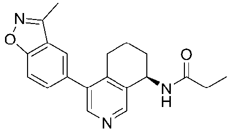 | 19B | 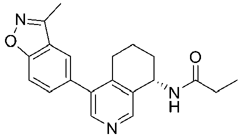 |
| 20 | 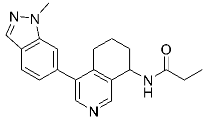 | 20A | 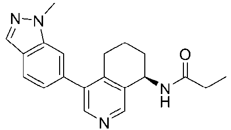 | 20B | 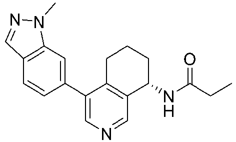 |
| 22 | 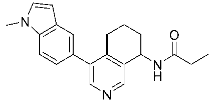 | 22A | 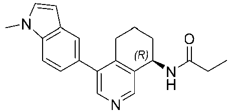 | 22B | 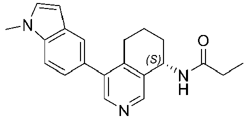 |
| 23 | 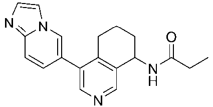 | 23A | 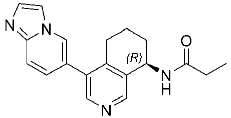 | 23B | 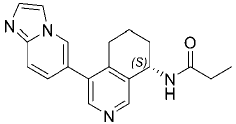 |
| 24 | 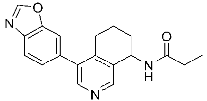 | 24A | 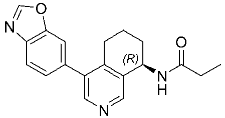 | 24B | 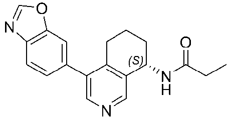 |
| 25 | 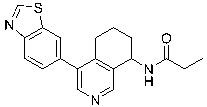 | 25A | 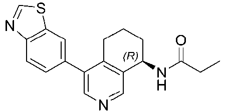 | 25B | 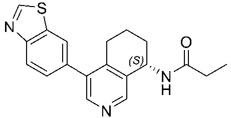 |
| 26 | 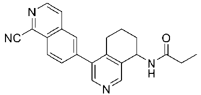 | 26A | 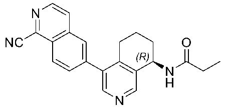 | 26B | 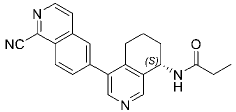 |
| 27 | 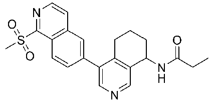 | 27A | 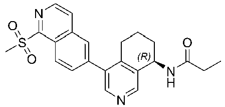 | 27B | 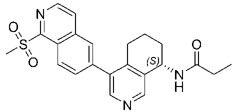 |
| 28 | 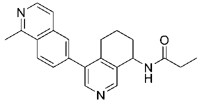 | 28A | 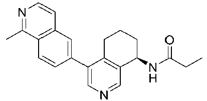 | 28B | 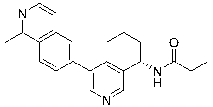 |
| 29 | 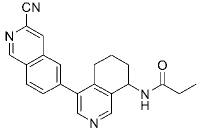 | 29A | 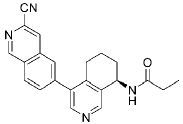 | 29B | 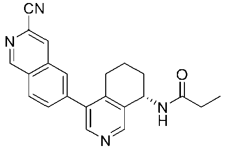 |
| 31 | 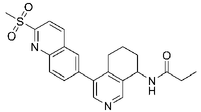 | 31A | 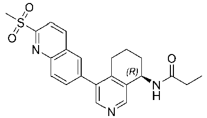 | 31B | 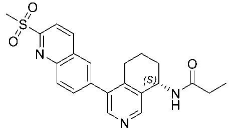 |
| 32 | 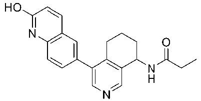 | 32A | 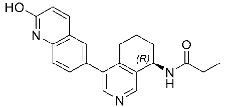 | 32B | 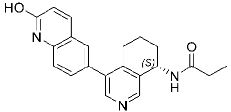 |
| 33 | 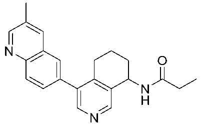 | 33A | 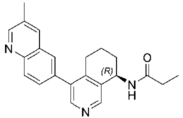 | 33B | 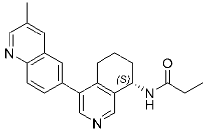 |
| 34 | 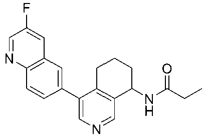 | 34A | 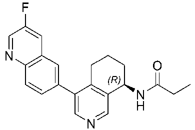 | 34B | 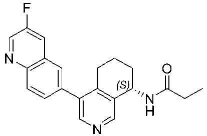 |
| 35 | 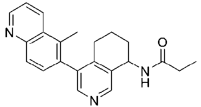 | 35A | 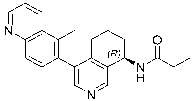 | 35B | 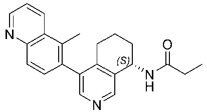 |
| 36 | 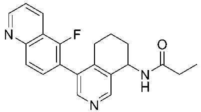 | 36A | 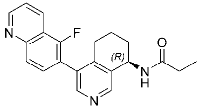 | 36B | 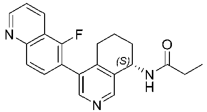 |
| 37 | 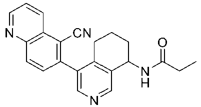 | 37A | 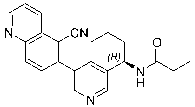 | 37B | 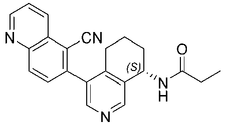 |
| 38 | 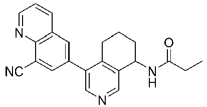 | 38A | 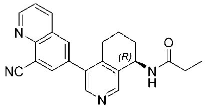 | 38B | 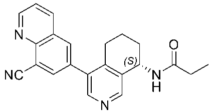 |
| 39 | 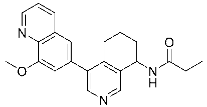 | 39A | 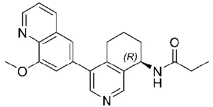 | 39B | 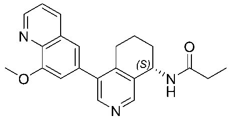 |
| 41 | 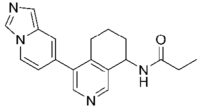 | 41A | 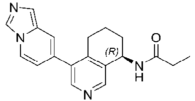 | 41B | 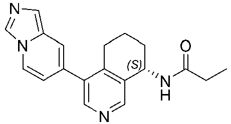 |
| 42 | 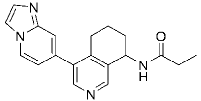 | 42A | 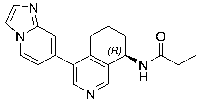 | 42B | 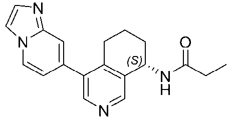 |
| 43 | 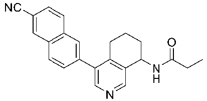 | 43A | 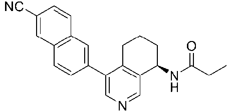 | 43B | 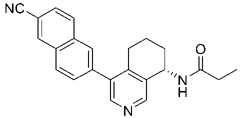 |
| 44 | 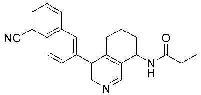 | 44A | 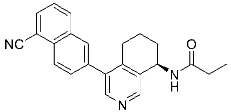 | 44B | 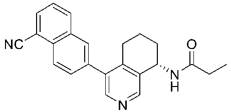 |
| 45 | 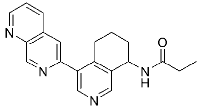 | 45A | 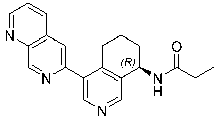 | 45B | 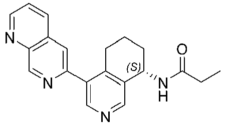 |
| 46 | 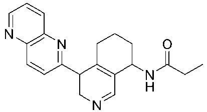 | 46A | 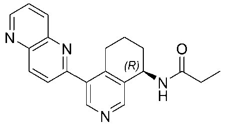 | 46B | 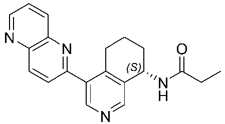 |
| 47 | 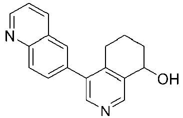 | 47A | 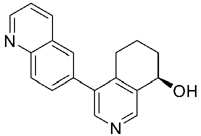 | 47B | 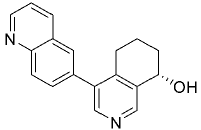 |
| 48 | 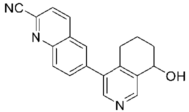 | 48A | 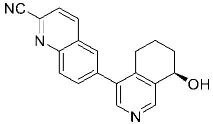 | 48B | 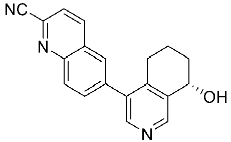 |
| 49 . | 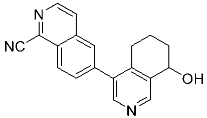 | 49A | 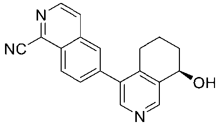 | 49B | 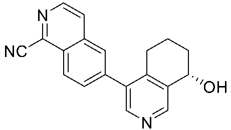 |

7. The compound, an isomer thereof, a racemate thereof, or a pharmaceutically acceptable salt thereof according to any one of claims 1-6, **characterized in that** one or more hydrogen atoms of the compound are substituted by the isotope deuterium (²H).

8. The compound, an isomer thereof, a racemate thereof, or a pharmaceutically acceptable salt thereof according to claim 7, **characterized in that** the compound substituted by deuterium is selected from the following structures:
| No. | Structure | No. | Structure | No. | Structure |
|---|---|---|---|---|---|
| 40 | 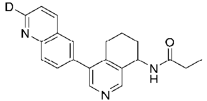 | 40A | 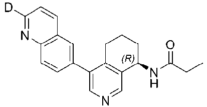 | 40B | 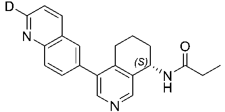 |

9. A pharmaceutical composition, **characterized in that** the pharmaceutical composition comprises the compound, an isomer thereof, a racemate thereof, or a pharmaceutically acceptable salt thereof according to any one of claims 1-8, and one or more pharmaceutically acceptable excipients and/or carriers.

10. Use of the compound, an isomer thereof, a racemate thereof, or a pharmaceutically acceptable salt thereof according to any one of claims 1-8, or the pharmaceutical composition according to claim 9 in the preparation of a medicament for treating or preventing diseases associated with elevated activity level of CYP11B2.

11. The use according to claim 10, **characterized in that** the diseases associated with elevated activity level of CYP11B2 are selected from hypertension, chronic kidney disease, primary aldosteronism, diabetic nephropathy, congestive heart failure or Cushing's syndrome.
